# EUROPEAN PATENT SPECIFICATION

(11) **EP 3 537 878 B1**
(45) Date of publication and mention of the grant of the patent: **22.11.2023**
(21) Application number: 17868136.7
(22) Date of filing: 18.05.2017
(51) Int. Cl.: A01N 25/12, A01N 25/14, A01N 25/02, A01N 25/04, A01N 25/06, A01N 25/18, C09D 5/16, A01N 63/22, A01N 63/25, A01N 63/28, A01N 63/00, A01P 13/00

(54) **ALGICIDAL ORGANISMS**
ALGIZIDE ORGANISMEN
ORGANISMES ALGICIDES

(30) Priority: 03.11.2016 US 201662416796 P
(43) Date of publication of application: 18.09.2019
(73) Proprietor: Pro Farm Group, Inc., Davis, CA 95618 (US)
(72) Inventor: MALIN, John, Davis, CA 95618 (US); CORDOVA-KREYLOS, Ana, Lucia, Davis, CA 95618 (US); MARRONE, Pamela, Davis, CA 95618 (US)
(74) Representative: Hertin und Partner Rechts- und Patentanwälte PartG mbB
(86) International application number: PCT/US2017/033309
(87) International publication number: WO 2018/084895

(56) References cited:
- WO-A1-2016/039961
- WO-A2-2008/124675
- KR-A- 19980 065 146
- KR-B1- 100 468 053
- US-A1- 2014 221 207
- ZHANG, B. ET AL.: 'Streptomyces alboflavus RPS and its novel and high algicidal activity against harmful algal bloom species Phaeocystis globosa' PLOS ONE vol. 9, no. 3, 27 March 2014, pages 1 - 10, XP055481335

## Description

### CROSS-REFERENCE TO RELATED APPLICATIONS

This application claim priority to U.S. provision application serial number 62/416,796 filed on November 3, 2016 under 35 U.S.C. 119(e).

### TECHNICAL FIELD OF THE INVENTION

The present disclosure relates to compositions and methods for controlling various algae.

### BACKGROUND OF THE INVENTION

Toxic algae blooms have been widespread in lakes, reservoirs, and rivers throughout the world. The blooms cause livestock death and threaten human health and aquatic ecosystems. Although several approaches have been proposed to eliminate the blooms, those approaches were hardly successful. There is no complete way to remove them. Therefore, there is a need for various approaches to devise multiple strategies.

### SUMMARY OF THE INVENTION

Water blooms have become a worldwide environmental problem. Recently, algicidal bacteria have attracted wide attention as possible agents for inhibiting algal water blooms. For example, US2014/221207A1 discloses compositions comprising a *Burkholderia* strain as an active ingredient, which can have algicidal activity, wherein the composition may additionally comprise another microorganism, such as an agent derived from *Bacillus sp*., *Brevibacillus sp*., and *Streptomyces sp.* KR 100 468 053 B1, KR 1998 0065146 A and Zhang, B. et al in PLOS ONE, 2014, 9 (3), 1-10 entitled "Streptomyces alboflavus RPS and its novel and high algicidal activity against harmful algal bloom species Phaeocystis globosa" all teach the algicidal effects of some *Streptomyces* strains.

Provided are isolated compounds and compositions derived from *Beauveria bassiana* O379 (NRRL-67406), *Brevibacillus brevis* HNYM3, O989 (NRRL-B67408), *Streptomyces spororaveus* F18, P842 (NRRL-B67410), *Paenibacillus elgii* SD17, P858 (NRRL-B67407), or *Streptomyces* sp. Q004 (NRRL-67409) fermentation having algicidal properties.

Specifically, the present invention relates to an algicidal composition comprising: a whole cell broth, filtrate, supernatant, or extract collected from Beauveria bassiana 0379 (NRRL- 67406), Brevibacillus brevis HNYM3, 0989 (NRRL-B67408), Streptomyces spororaveus F18, P842 (NRRL-B67410), Paenibacillus elgii SD17, P858 (NRRL-B67407), or Streptomyces sp. Q004 (NRRL-67409) fermentation.

Furthermore, the invention relates to a method for inhibiting the growth of an algae comprising the steps of: introducing the algicidal composition of the invention in a location where inhibition is desired, in amounts effective for inhibiting said algae.

In a particular aspect, fermentation products from such organisms can modulate or inhibit one or more species of algae such as *Chlamydomonas reinhardtii, Pesudokirchneriella subacpitata*, *Scenedesmus quadricata*, *Anabaena* sp., *Oscillatoria tenuis*, *Microcystis aeruginosa*, *Navicula* sp., *Spirogyra* sp., or *Chara* sp.

Further provided is a method of modulating or inhibiting at least one species of an algae in a location with an amount of a cell suspension or whole cell broth having cells derived from one or more of the following species: *Beauveria bassiana* O379 (NRRL-67406), *Brevibacillus brevis* HNYM3, O989 (NRRL-B67408), *Streptomyces spororaveus* F18, P842 (NRRL-B67410), *Paenibacillus elgii* SD17, P858 (NRRL-B67407), or *Streptomyces* sp. Q004 (NRRL-67409), wherein said one more species produce one or more substances that control algae, or a supernatant, filtrate, cell fraction, one or more metabolites, one or more compounds and/or extract derived therefrom, effective to modulate said algae such as *Chlamydomonas reinhardtii*, *Pesudokirchneriella subacpitata*, *Scenedesmus quadricata*, *Anabaena* sp., *Oscillatoria tenuis*, *Microcystis aeruginosa*, *Navicula* sp., *Spirogyra* sp., or *Chara* sp.

In an aspect, the present disclosure relates to applying a whole cell broth, supernatant, filtrate, or cell fraction in a location in an amount effective to control algae infestation. The location can be a body of water or in paint, or on concrete, pavement, tile, porcelain, vinyl, brick, stucco, fiberglass, and aluminum surfaces. These surfaces may be painted or unpainted. Generally, the algaecide is applied to the surface until it is wet or until runoff is observed.

Further, the composition set forth above can be applied in combination with an antibiotic or anti-fungal agent, particularly an antibiotic effective against soil-borne bacteria. Described, but not forming part of the invention, is a combination comprising the substances set forth above and another antibiotic effective against soil-borne bacteria. These combinations can also be compositions. Described, but not forming part of the invention, is the use of these substances and antibiotics in formulating these combinations.

Even further, the composition set forth above can be formulated as dustable powders (DP), soluble powders (SP), water soluble granules (SG), water dispersible granules (WG), wettable powders (WP), granules (GR) (slow or fast release), soluble concentrates (SL), oil miscible liquids (OL), ultra-low volume liquids (UL), emulsifiable concentrates (EC), dispersible concentrates (DC), emulsions (both oil in water (EW) and water in oil (EO)), micro-emulsions (ME), suspension concentrates (SC), oil-based suspension concentrate (OD), aerosols, fogging/smoke formulations, capsule suspensions (CS) and seed treatment formulations.

### BRIEF DESCRIPTION OF THE DRAWINGS

For a more complete understanding of the features and advantages of the present disclosure, reference is now made to the detailed description of the invention along with the accompanying figures and in which:
FIGURE 1 denotes dose responses of *Beauveria bassiana* O379 (NRRL-67406) on *Pseucokirchneriella subcapitata* in 96 well plates.
FIGURE 2 denotes dose responses of *Beauveria bassiana* O379 (NRRL-67406) on *Anabaena* sp. in a 96 well plate.
FIGURE 3 denotes dose response of *Beauveria brevis* HNYM3, 0989 (NRRL-B67408) on *Pseucokirchneriella subcapitata* in 96 well plates.
FIGURE 4 denotes dose response of *Beauveria brevis* HNYM3, 0989 (NRRL-B67408) on *Anabaena* sp. in a 96 well plate.
FIGURE 5 denotes dose response of *Streptomyces spororaveus* F18, P842 (NRRL-B67410) on *Pseucokirchneriella subcapitata* in 96 well plates
FIGURE 6 denotes dose response of *Streptomyces spororaveus* F18, P842 (NRRL-B67410) on *Anabaena* sp. in a 96 well plate.
FIGURE 7 denotes dose response of *Paenibacillus elgii* SD17, P858 (NRRL-B67407) on *Pseucokirchneriella subcapitata* in 96 well plates.
FIGURE 8 denotes dose response of *Paenibacillus elgii* SD17, P858 (NRRL-B67407) on *Anabaena* sp. in a 96 well plate.
FIGURE 9 denotes dose response of *Streptomyces* sp. Q004 (NRRL-67409) on *Pseucokirchneriella subcapitata* in 96 well plates.
FIGURE 10 denotes dose response of *Streptomyces* sp. Q004 (NRRL-67409) on *Anabaena* sp. in a 96 well plate.

### DETAILED DESCRIPTION OF THE INVENTION

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. However, the invention is defined by the claims.

It must be noted that as used herein and in the appended claims, the singular forms "a," "and" and "the" include plural references unless the context clearly dictates otherwise. For example, "a substance" also encompasses "substances".

As defined herein, the term "modulate" is used to mean to alter the amount of algae infestation or rate of spread of algae infestation, or kill, be lethal or toxic to algae in a location. "Modulate algae infestation" also encompasses modulating the effects of said infestation.

As used herein "algae" refers to any of various chiefly aquatic, eukaryotic, photosynthetic organisms, ranging in size from single-celled forms to the giant kelp. The term may further refer to photosynthetic protists responsible for much of the photosynthesis on Earth. As a group, the algae are polyphyletic. Accordingly, the term may refer to any protists considered to be algae from the following groups, alveolates, chloraraachniophytes, cryptomonads, euglenids, glaucophytes, haptophytes, red algae such as Rhodophyta, stramenopiles, and viridaeplantae. The term refers to the green, yellow-green, brown, and red algae in the eukaryotes. The term can also refer to the cyanobacteria in the prokaryotes. The term also refers to green algae, blue algae, and red algae. In one embodiment, the algae is filamentous or planktonic greens or cyanobacteria.

As defined herein, "derived from" and "obtainable from" means directly isolated or obtained from a particular source or alternatively having identifying characteristics of a substance or organism isolated or obtained from a particular source. These terms are used interchangeably throughout the specification.

As defined herein, "derived from *Beauveria bassiana* O379 (NRRL-67406), *Brevibacillus brevis HNYM3,* O989 *(NRRL-B67408), Streptomyces spororaveus* F18, P842 (NRRL-B67410), *Paenibacillus elgii* SD17, P858 (NRRL-B67407), or *Streptomyces sp.* Q004 (NRRL-67409)" means a cell broth comprising cells from *Beauveria bassiana* O379 (NRRL-67406), *Brevibacillus brevis* HNYM3, O989 (NRRL-B67408), *Streptomyces spororaveus* F18, P842 (NRRL-B67410), *Paenibacillus elgii* SD17, P858 (NRRL-B67407), or *Streptomyces* sp. Q004 (NRRL-67409), a cell suspension comprising cells from a *Beauveria bassiana* O379 (NRRL-67406), *Brevibacillus brevis* HNYM3, O989 (NRRL-B67408), *Streptomyces spororaveus* F18, P842 (NRRL-B67410), *Paenibacillus elgii* SD17, P858 (NRRL-B67407), or *Streptomyces* sp. Q004 (NRRL-67409) as well as a cell fraction, supernatant, filtrate, or extract collected from fermentation. The extract can be derived from not only a cell suspension or whole cell broth but also a filtrate, supernatant or fraction derived from said whole cell broth or cell suspension.

As defined herein, an "isolated compound" is essentially free of other compounds or substances, e.g., at least about 20% pure, preferably at least about 40% pure, more preferably about 60% pure, even more preferably about 80% pure, most preferably about 90% pure, and even most preferably about 95% pure, as determined by analytical methods, including but not limited to chromatographic methods, electrophoretic methods.

As defined herein, "whole broth culture" or "whole cell broth" refers to a liquid culture containing both cells and fermentation media. A whole cell broth can also be obtained from bacteria grown on a plate, by harvesting the cells from the plate and suspending them in water or other liquid. The terms "whole broth culture" and "whole cell broth" are used interchangeably.

As defined herein, "supernatant" refers to the liquid remaining when cells grown in broth or harvested in another liquid from an agar plate and are removed by centrifugation, filtration, sedimentation, or other means known in the art.

As defined herein, "filtrate" refers to liquid from a whole broth culture that has passed through a membrane.

As defined herein, "extract" refers to a liquid substance removed from cells by a solvent (water, detergent, buffer, organic solvent) and separated from the cells by centrifugation, filtration, phase partition or other method.

As defined herein, "inactivated" microorganism refers to dead microbes, or microbes not in the active growing stage (e.g., spores). Non-limiting ways of inactivation include heat-killed or chemical killed. In one embodiment, the microbes can be lysed.

The compositions of the present disclosure are derived from *Beauveria bassiana* O379 (NRRL-67406), *Brevibacillus* brevis HNYM3, O989 (NRRL-B67408), *Streptomyces spororaveus* F18, P842 (NRRL-B67410), *Paenibacillus elgii* SD17, P858 (NRRL-B67407), or *Streptomyces* sp. Q004 (NRRL-67409). The composition can be a cell suspension or whole cell broth and also a filtrate, supernatant or fraction derived from said whole cell broth or cell suspension.

As noted above, the compositions are derived from an organism having the identifying characteristics of *Beauveria bassiana* O379 (NRRL-67406), Brevibacillus *brevis* HNYM3, O989 (NRRL-B67408), *Streptomyces spororaveus* F18, P842 (NRRL-B67410), *Paenibacillus elgii* SD17, P858 (NRRL-B67407), or *Streptomyces* sp. Q004 (NRRL-67409). Methods not according to the invention comprise cultivating these organisms and optionally obtaining the compounds by isolating these compounds from the cells of these organisms.

In particular, the organisms are cultivated in a nutrient medium using methods known in the art. The organisms can be cultivated by shake flask cultivation, small scale or large scale fermentation (including but not limited to continuous, batch, fed-batch, or solid state fermentations) in laboratory or industrial fermentors performed in suitable medium and under conditions allowing cell growth. The cultivation may take place in suitable nutrient medium comprising carbon and nitrogen sources and inorganic salts, using procedures known in the art. Suitable media are available may be available from commercial sources or prepared according to published compositions. A particular embodiment is disclosed in the examples *infra* and in US Patent No. 6 194 194.

After cultivation, the cells can be concentrated and subsequently suspended in a buffer to obtain a cell suspension. A suspension of dead cells may be used. Live cells in the cellular suspension may be killed by at least one of the following: irradiating, heating, drying, or treating cells with other chemical of physical means. A dead cell suspension is not required for activity against algae species.

Substances which modulate and in particular are toxic to algae can be extracted from the suspension. The extract can be fractionated by chromatography. Chromatographic fractions can be assayed for toxic activity against algae, such as *Chlamydomonas reinhardtii, Pesudokirchneriella subacpitata, Scenedesmus quadricata, Anabaena sp., Oscillatoria tenuis, Microcystis aeruginosa, Navicula sp., Spirogyra sp*., or *Chara sp.* using methods known in the art; one particular method is disclosed in the examples, *infra.* This process can be repeated one or more times using the same or different chromatographic methods.

In one embodiment, compositions can comprise whole cell broth cultures, liquid cultures, or suspensions collected from *Beauveria bassiana* O379 (NRRL-67406), *Brevibacillus brevis* HNYM3, O989 (NRRL-B67408), *Streptomyces spororaveus* F18, P842 (NRRL-B67410), *Paenibacillus elgii* SD17, P858 (NRRL-B67407), or *Streptomyces sp.* Q004 (NRRL-67409) fermentation.

The compositions set forth above can be formulated in any manner. Formulation examples include, but are not limited to, Emulsifiable concentrates (EC), Wettable powders (WP), soluble liquids (SL), Aerosols, Ultra-low volume concentrate solutions (ULV), Soluble powders (SP), Microencapsulation, Water dispersed Granules, Flowables (FL), Microemulsions (ME), Nano-emulsions (NE), etc. In any formulation described herein, percent of the active ingredient is within a range of 0.01% to 99.99%. Another embodiment is non-formulated whole cell broth, or concentrated whole-cell-broth.

The compositions can also be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra-low volume liquids (UL), emulsifiable concentrates (EC), dispersible concentrates (DC), emulsions (both oil in water (EW) and water in oil (EO)), micro-emulsions (ME), suspension concentrates (SC), oil-based suspension concentrate (OD), aerosols, fogging/smoke formulations, capsule suspensions (CS) and seed treatment formulations. The formulation type chosen in any instance will depend upon the particular purpose envisaged and the physical, chemical and biological properties of the microbes.

In another embodiment, the compositions can be in the form of a liquid, gel or solid. A solid composition can be prepared by suspending a solid carrier in a solution of active ingredient(s) and drying the suspension under mild conditions, such as evaporation at room temperature or vacuum evaporation at 65°C or lower. A composition can comprise gel-encapsulated active ingredient(s). Such gel-encapsulated materials can be prepared by mixing a gel-forming agent (e.g., gelatin, cellulose, or lignin) with a culture or suspension of live or inactivated microorganism(s) or a cell-free filtrate or cell fraction of a culture or suspension, or a spray- or freeze-dried culture, cell, or cell fraction or in a solution of algicidal compounds used in the method of the invention; and inducing gel formation of the agent.

Yet in another embodiment, the composition can additionally comprise a surfactant to be used for the purpose of emulsification, dispersion, wetting, spreading, integration, disintegration control, stabilization of active ingredients, and improvement of fluidity or rust inhibition. In a particular embodiment, the surfactant is a non-phytotoxic non-ionic surfactant which preferably belongs to Environmental Protection Agency (EPA) List 4B. In another particular embodiment, the nonionic surfactant is polyoxyethylene (20) monolaurate. The concentration of surfactants may range between 0.1-35% of the total formulation, preferred range is 5-25%. The choice of dispersing and emulsifying agents, such as non-ionic, anionic, amphoteric and cationic dispersing and emulsifying agents, and the amount employed is determined by the nature of the composition and the ability of the agent to facilitate the dispersion of the compositions of the present invention.

The compositions can also include, but are not limited to, aminoglycoside antibiotics which include a number of molecules (e.g. kanamycin, neomycin, gentamycin, derivative G418 and paromycin) which are toxic to plant, fungal and animal cells (Nap et al. 1992) as well as bacterial neomycin phosphotransferase II and aerocyanidin, aerocavin, 3,6-dihydroxy-indoxazene, and monobactam SB-26.180.

In one embodiment, the compositions and substances set forth above can be used to modulate or inhibit the amount of algae infestation in a location such as a closed or open body of water, or a on a solid surface such as plastic, concrete, wood, fiberglass, pipes made of iron and polyvinyl chloride, surfaces covered with coating materials and/or paints.

In another embodiment, the compositions inhibit the growth of algae such as *Chlamydomonas reinhardtii, Pesudokirchneriella subacpitata, Scenedesmus quadricata, Anabaena sp., Oscillatoria tenuis, Microcystis aeruginosa, Navicula sp., Spirogyra sp., Chara sp,* or any combinations thereof

Application of an effective algicidal control by a supernatant, filtrate or extract containing active metabolite, or compound produced by or obtained or derived from a supernatant, filtrate or extract of *Beauveria bassiana* O379 (NRRL-67406), *Brevibacillus brevis* HNYM3, O989 (NRRL-B67408), *Streptomyces spororaveus* F18, P842 (NRRL-B67410), *Paenibacillus elgii* SD17, P858 (NRRL-B67407), or *Streptomyces* sp. Q004 (NRRL-67409) is described. The strain or supernatant or filtrate or extract, metabolite and/or compound are applied, alone or in combination with another algicidal substance, in an effective algae control. An effective amount is defined as those quantities of microorganism cells, supernatant, filtrate or extract, metabolite and/or compound alone or in combination with or without another algicidal substance that is sufficient to modulate algae infestation. The effective rate can be affected by algae species present, stage of pest growth, pest population density, and environmental factors such as temperature, wind velocity, rain, time of day and seasonality. The amount that will be within an effective range in a particular instance can be determined by laboratory or field tests such as the one listed in the present examples.

Yet in another embodiment, the composition set forth herein can also be combined with another microorganism and/or pesticide (*e*.*g*,, fungicide, algaecide). Other microorganism can include, but is not limited to, an agent derived from *Bacillus* sp. (e.g., *Bacillus firmus*, *Bacillus thuringiensis, Bacillus pumilus, Bacillus licheniformis*, *Bacillus amyloliquefaciens*, *Bacillus subtilis*), *Paecilomyces* sp. (*P. lilacinus*), *Pasteuria* sp. (*P*. *penetrans*), *Pseudomonas* sp., *Brevabacillus* sp., *Lecanicillium* sp., *Ampelomyces* sp., *Pseudozyma* sp., *Streptomyces* sp. (*S*. *bikiniensis*, *S. costaricanus*, *S. avermitilis*), *Burkholderia* sp., *Trichoderma* sp., *Gliocladium* sp., *avermectin*, *Myrothecium* sp., *Paecilomyces* spp., *Sphingobacterium* sp., *Arthrobotrys* sp., *Chlorosplrnium*, *Neobulgaria*, *Daldinia*, *Aspergillus*, *Chaetomium*, *Lysobacter* spp, *Lachnum papyraceum*, *Verticillium suchlasporium, Arthrobotrys oligospora, Verticillium chlamydosporium, Hirsutella rhossiliensis, Pochonia chlamydosporia, Pleurotus ostreatus, Omphalotus olearius, Lampteromyces japonicas*, *Brevudimonas* sp., or *Muscodor* sp.

In another embodiment, the composition can also be used in combination (such as in a compositional mixture, or a simultaneous or sequential application) with one or more of the following: 3-(4-chloro-2,6-dimethylphenyl)-4-hydroxy-8-oxa-1-azaspiro[4,5]dec-3-en-2-one; 3-(4'-chloro-2,4-dimethyl[1,1'-biphenyl]-3-yl)-4-hydroxy-8-oxa-1-azaspiro-[4,5]dec-3-en-2-one; 4-[[(6-chloro-3-pyridinyl)methyl]methylamino]-2(5H)-furanone; 4-[[(6-chloro-3-pyridinyl)methyl]cyclopropylamino]-2(5H)-furanone; 3-chloro-N2-[(1S)-1-methyl-2-(methylsulfonyl)ethyl]-N1-[2-methyl-4-[1,2,2,2-tetrafluoro-1-(trifluoromethyl) ethyl] phenyl]-1,2-benzenedicarboxamide; 2-cyano-N-ethyl-4-fluoro-3-methoxy-benenesulfonamide; 2-cyano-N-ethyl-3-methoxy-benzenesulfonamide; 2-cyano-3-difluoromethoxy-N-ethyl-4-fluoro-benzenesulfonamide; 2-cyano-3-fluoromethoxy-N-ethyl-benzenesulfonamide; 2-cyano-6-fluoro-3-methoxy-N,N-dimethyl-benzenesulfonamide; 2-cyano-N-ethyl-6-fluoro-3-methoxy-N-methyl-benzenesulfonamide; 2-cyano-3-difluoromethoxy-N,N-dimethylbenzenesulfon-amide; 3-(difluoromethyl)-N-[2-(3,3-dimethylbutyl)phenyl]-1-methyl-1H-pyrazole-4--carboxamide; N-ethyl-2,2-dimethylpropionamide-2-(2,6-dichloro-α,α,α,-trifluoro-p-tolyl) hydrazone; N-ethyl-2,2-dichloro-1-methylcyclopropane-carboxamide-2-(2,6-dichloro-α,α,α,-trifluoro-p-tolyl) hydrazone nicotine; O-{(E-)-[2-(4-chloro-phenyl)-2-cyano-1-(2-trifluoromethylphenyl)-vinyl]}S-methyl thiocarbonate; (E)-N1-[(2-chloro-1,3-thiazol-5-ylmethyl)]-N2-cyano-N1-methylacetamidine; 1-(6-chloropyridin-3-ylmethyl)-7-methyl-8-nitro-1,2,3,5,6,7-hexahydro -imidazo[1,2-a]pyridin-5-ol; 4-[4-chlorophenyl-(2-butylidine-hydrazono)methyl)]phenyl mesylate; and N-Ethyl-2,2-dichloro-1-methylcyclopropanecarboxamide-2-(2,6-dichloro-α,α,α,-trifluoro-p-tolyl)hydrazone.

In the present disclosure, the following surfactant can be used together with the above-mentioned microorganism fermentation to promote emulsification, dispersion, solubilization and permeation.

Non-limiting examples of nonionic surfactants include sorbitan fatty acid esters, polyoxyalkylene sorbitan fatty acid esters, polyoxyalkylene fatty acid esters, glycerol fatty acid esters, polyoxyalkylene glycerol fatty acid esters, polyglycerol fatty acid esters, polyoxyalkylene polyglycerol fatty acid esters, sucrose fatty acid esters, resin acid esters, polyoxyalkylene resin acid esters, polyoxyalkylene alkyl ethers, polyoxyalkylene alkylphenyl ethers, alkyl(poly)glycosides and polyoxyalkylenealkyl(poly)glycosides. Preferably, an ether group-containing nonionic surfactant having no nitrogen atom and ester group-containing nonionic surfactant can be used.

Non-limiting examples of anionic surfactants include carboxylic, sulfonic, sulfuric ester group-containing and phosphoric ester group-containing surfactants, and a carboxylic and phosphoric ester group-containing surfactants.

Non-limiting examples of the carboxylic surfactants include fatty acids having 6-30 carbon atoms or salts thereof, polyhydric carboxylic acid salts, polyoxyalkylene alkyl ether carboxylic acid salts, polyoxyalkylene alkylamide ether carboxylic acid salts, rhodinic acid salts, dimmer acid salts, polymer acid salts and tall oil fatty acid salts.

Non-limiting examples of the sulfonic surfactants include alkylbenezenesulfonic acid salts, alkylsulfonic acid salts, alkylnaphthalenesulfonic acid salts, naphthalenesulfonic acid salts, diphenyl ether sulfonic acid salts, condensates of alkylnaphthalenesulfonic acid, and condensate of naphthalenesulfonic acid.

Non-limiting examples of the sulfuric ester group-containing surfactants include alkylsulfuric ester salts (alkylsulfuric acid salts), polyoxyalkylene alkylsulfuric ester salts (polyoxyalkylene alkylsulfuric acid salts), polyoxyalkylene alkyl phenyl ether sulfuric acid salts, tristyrenatedphenol sulfuric acidester salts, polyoxyalkylene distyrenated phenol sulfuric acid ester salts and alkylpolyglycoside sulfuric acid salts.

Non-limiting examples of phosphoric acid ester group-containing surfactants include alkyl phosphoric acid ester salts, alkylphenylphosphoric acid ester salts, polyoxyalkylene alkylphosphoric acid ester salts and polyoxyalkylene alkylpheneylphosphoric acid ester salts.

Non-limiting examples of the salts include metallic salts (such as salts of Na, K, Ca, Mg and Zn), ammonium salts, alkanol amine salts and aliphatic amine salts.

Non-limiting examples of amphoteric surfactants include amino acid group-containing, betaine group-containing, imidazoline group-containing and amine oxide group-containing surfactants.

Non-limiting examples of the amino acid group-containing surfactants include acylamino acid salts, acylsarcosine acid salts, acyloylmethylaminopropionic acid salts, alkylaminopropionic acid salts and acylamide ethylhydroxyethylmethylcarboxylic acid salts.

Non-limiting examples of the betaine group-containing surfactants include alkyldimethylbetaine, alkylhydroxyethylbetaine, acylamide propylhydroxypropylammonia sulfobetaine, acylamide propylhydroxypropylammonia sulfobetaine and ricinoleic acid amide propyl dimethylcarboxy methylammonia betaine.

Non-limiting examples of the imidazoline group-containing surfactants include alkylcarboxy methylhydroxy ethylimidazolinium betaine and alkylethoxy carboxy methylimidazolinium betaine.

Non-limiting examples of the amine oxide group-containing surfactants include alkyldimethylamine oxide, alkyldiethanolamine oxide and alkylamidepropylamine oxide.

One kind of the above-mentioned surfactants may be used, and a mixture of two or more kinds of the above-mentioned surfactants can be used. In the case that one of these surfactants comprises a polyoxyalkylene group, the polyoxyalkylene group can be a polyoxyethylene group and the average mole number of added polyoxyethylene groups can be from 1 to 50.

As the surfactant, at least one compound selected from ester group-containing nonionic surfactants, ether group-containing nonionic surfactants having no nitrogen atom, amphoteric surfactants, carboxylic anionic surfactants and phosphoric anionic surfactant can be used.

The following fertilizer components may be used together with the above-mentioned microorganism fermentation. Specific examples thereof can be inorganic or organic compounds which can supply elements such as N, P, K, Ca, Mg, S, B, Fe, Mn, Cu, Zn, Mo, Cl, Si and Na, in particular N, P, X, Ca and Mg. Examples of such inorganic compounds include ammonium nitrate, potassium nitrate, ammonium sulfate, ammonium chloride, ammonium phosphate, sodium nitrate, urea, ammonium carbonate. potassium phosphate, calcium superphosphate, fused phosphate fertilizer (3MgO.CaO.P₂O₅.3CaSiO₂), potassium sulfate, potassium chloride, nitrate of lime, slaked lime, carbonate of lime, magnesium sulfate, magnesium hydroxide and magnesium carbonate. Examples of the organic compounds include fowl droppings, cow dung, Bark compost, amino acid, peptone, amino acid solution, fermentation extracts, calcium salts of organic acids (such as citric acid, gluconic acid and succinic acid), and calcium salts of fatty acids (such as formic acid, acetic acid, propionic acid, caprylic acid, capric acid and caproic acid). These fertilizer components may be used together with the surfactant. In the case that fertilizer components are sufficiently applied as basal fertilizer to soil as seen in outdoor cultivation of a rice-plant or vegetables, it is unnecessary to mix the fertilizer components.

### EXAMPLES

The composition and methods set forth herein will be further illustrated in the following, non-limiting Examples. The examples are illustrative of various embodiments only and do not limit the claimed invention regarding the materials, conditions, weight ratios, process parameters and the like recited herein.

### Example 1.

*Beauveria bassiana* O379 (NRRL-67406) is a fungus that was discovered as an initial hit on *Pesudokirchnneriella subcapitata* and its activity was verified on both *Pesudokirchnneriella subcapitata* and *Chamydomonas reinhardtii.* The microbe was identified by its ITS sequence as *Beauveria bassiana* O379 (NRRL-67406). *Beauveria bassiana* O379 (NRRL-67406) has the broadest activity of any algaecide verified hit. It has activity on all the species that it has been screened on, and it has greater than 75% activity on all species except *Microcystis aeruginosa.* However, LC50s for *Beauveria bassiana* O379 (NRRL-67406) are somewhat higher than the other top hits, particularly for Green Algae, shown in Table 1.

When tested in a system that simulates an algae infested rice field, *Beauveria bassiana* O379 (NRRL-67406) had moderate control of *Anabaena sp*., but no control of overall Chlorophyll A content.

**Table 1 Beauveria bassiana O379 (NRRL-67406) Spectrum and LC50 Summary**

| **Algae Type** | **Spectrum Species** | **Activity** | **LC 50 (%v/v)** |
|---|---|---|---|
| Green Algae | *Chlamydomonas reinhardtii* | Active | 2.5% - 5% |
| | *Pesudokirchneriella subacpitata* | Active | 4% - 4.4% |
| | *Scenedesmus quadricata* | Active | |
| Blue-Green Algae | *Anabaena sp.* | Active | 1.2% - 1.6% |
| | *Oscillatoria tenius* | Active | |
| | *Microcystis aeruginosa* | Somewhat Active | |
| Diatom | *Navicula sp.* | Active | |
| Macroalgae | *Chara sp.* | Active | 2.5% - 5% |
| | *Spirogyra sp.* | Active | 0.1% - 0.7% |

*Beauveria bassiana* O379 (NRRL-67406) was isolated and screened and identified as an initial hit. The activity was verified on *Chlamydomonas reinhardtii.*

Initial screening was conducted by dosing 750ul of early log phase *Pesudokirchnneriella subcapitata* or *Chamydomonas reinhardtii* culture (Bristol's Medium or Modified Bold 3N Medium, respectively, pH 6.2) with 100µl of cell free supernatant in a 48 well plate. Two wells were dosed per treatment. The plates were incubated under full spectrum fluorescent lights for three days. Isolates screened on *Pesudokirchnneriella subcapitata* were scored by measuring the fluorescence of Chlorophyll A (Ex: 440nm, Em: 680mn) for each well. Any isolate that yields a 75% reduction in Chlorophyll A compared to an untreated control was considered a hit. Isolates screened on *Chlamydomonas reinhardtii* were scored visually by comparing the treated wells to the untreated wells. The isolates that resulted in wells that appeared 75% less green than the untreated wells were considered hits.

Identifying the Microbe species. DNA was extracted from *Beauveria bassiana* O379 (NRRL-67406) using the Qigen DNeasy Plant Mini Kit. PCR amplification was performed the following day, using the ITS1 and ITS4 primers. The presence of a band on an agarose gel was confirmed, and the PCR reaction was cleaned using the Mo Bio UltraClean PCR Clean-Up Kit and sent to Davis Sequencing. The sequences were aligned using BioEdit, and the resulting Consensus Sequence was searched on the BLAST nucleotide database. The microbe was identified as *Beauveria bassiana* O379 (NRRL-67406).

Spectrum Testing. Microalgae. *Beauveria bassiana* O379 (NRRL-67406) was tested on a broad spectrum of algae species. As part of the spectrum testing, *Beauveria bassiana* O379 (NRRL-67406) was tested on *Pesudokirchnerella subcapitata* after being moved from V8 Medium to MBI-1 Medium (M1). *Beauveria bassiana* O379 (NRRL-67406) has the broadest spectrum of activity of any of the algaecide verified hits. It is active on all species it has been tested on, though its activity on *Microcystis areuginosa* is less than 75% inhibition, shown in Table 2.

**Table 2 Beauveria bassiana O379 (NRRL-67406) Spectrum Activity on Microalgae**

| **Algae Type** | **Spectrum Species** | **% Inhibition** | | **Activity** |
|---|---|---|---|---|
| | | **1st** | **2nd** | |
| Green Algae | *Pesudokirchne riella subacpitata* | 92.6 | 93.4 | Active |
| | *Scenedesmus quadricata* | 99.4 | 99.4 | Active |
| Blue-Green | *Anabaena sp.* | 91.9 | 88.7 | Active |
| Algae | *Oscillatoria tenius* | 81.9 | 78.7 | Active |
| | *Microcystis aeruginosa* | 52.4 | 56.4 | Somewhat Active |
| Diatom | *Navicula sp.* | 94.4 | 87.6 | Active |

Spectrum testing on microalgal species was carried out in a manner very similar to the initial screening on *Pseudokirchneriella subcapitata.* Testing was carried out in 48 well plates with 100ul of cell free supernatant dosed into 750ul of algae culture. Rather than an initial test followed by two verifications, two separate fermentations were concurrently tested for spectrum testing. If greater than 75% inhibition was seen for each fermentation, the isolate was considered to be "Active" on that spectrum species. If one or both of the fermentations yielded between 50% and 75% inhibition, the isolate was considered to be "Somewhat Active" on the spectrum species.

Macro Algae. *Beauveria bassiana* O379 (NRRL-67406) was tested on two species of macroalgae, *Spirogyra sp.* and *Chara sp.* Tests were scored visually. Thee fermentations were tested on *Chara sp.* and scores of 4 or above were considered active. Shown in Table 3.One fermentation was tested *Spirogyra sp.* in four replicates. At least three of the four replicates must show strong activity to be considered a hit, shown in Table4.

**Table 3 Beauveria bassiana O379 (NRRL-67406) Spectrum Activity on Chara sp.**

| | **Verification** | **Score** | **Activity** |
|---|---|---|---|
| *Chara sp.* | 1 st | 4 | Active |
| | 2nd | 5 | Active |
| | 3rd | 6 | Active |

**Table 4 Beauveria bassiana O379 (NRRL-67406) Spectrum Activity on Spirogyra sp.**

| | **Score** | **Activity** |
|---|---|---|
| *Spirogyra sp.* | ++++ | Active |

Spectrum testing on *Chara sp.* was performed by placing a sprig of *Chara* in a 15ml falcon tube containing 9 milliliters of mussel water and 1ml of cell-free supernatant. After five days, the tubes were scored visually. Uniformly yellowed or browned sprigs were scored "2", partially yellowed or browned sprigs were scored "1", and healthy sprigs were scored "0". Three replicates were tested for each sample. The three scores were added together. A score of "4" or more was considered "Active". A score of "3" was considered "Somewhat Active". Only one fermentation was tested for *Spirogyra sp.* Strands of *Spirogyra* were transferred into 750ul of EREM medium in 48 well plates, then the plates were dosed with 100ul of cell-free supernatant, four reps per treatment. After three days the plates were scored visually for health of the strands. Isolates were considered active if all the strands died in three of the four replicates.

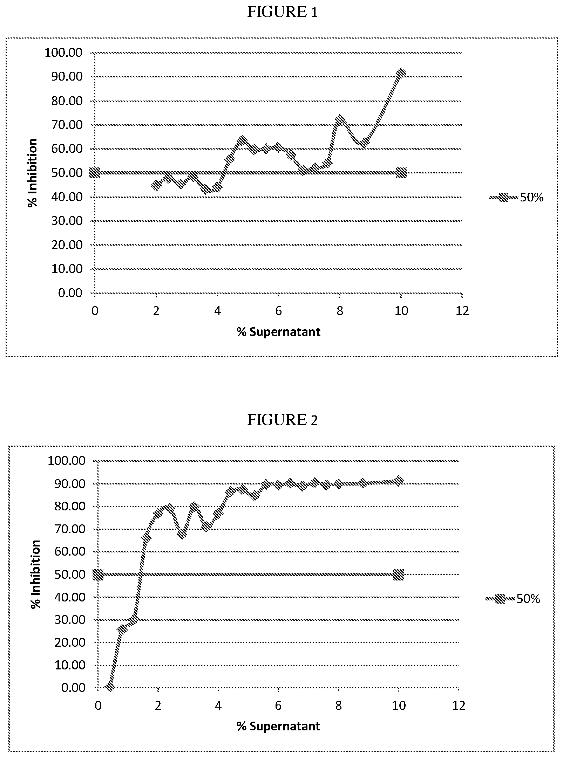

Dose Responses. Various dose responses of *Beauveria bassiana* O379 (NRRL-67406) cell-free supernatant were determined. Microalgae dose responses were performed using 96 well plates, shown in FIGURE 1 and FIGURE 2. Macroalgae dose responses were performed in either 48 well plates *(Spirogyra sp.)* or in 15ml falcon centrifuge tubes (*Chara sp*.), shown in Table 5 and Table 6.

**Table 5: Dose Response of Beauveria bassiana O379 (NRRL-67406) on Spirogyra sp. in a 48 well plate.**

| | **% *Beauveria bassiana* O379 (NRRL-67406) v/v** | | | | | | | |
|---|---|---|---|---|---|---|---|---|
| | **12** | **9.1** | **6.3** | **3.2** | **1.3** | **0.7** | **0.1** | **0** |
| ***Spirogyra sp.*** | + | + | +/- | +/- | +/- | +/- | - | - |
| | + | + | +/- | +/- | +/- | +/- | - | - |

**Table 6: Dose Response of Beauveria bassiana O379 (NRRL-67406) on Chara sp. in 15ml tubes.**

| | **% *Beauveria bassiana* O379 (NRRL-67406) v/v** | | |
|---|---|---|---|
| | **5%** | **2.50%** | **1%** |
| ***Chara sp.*** | Active | Not Active | Not Active |

Microalgae dose responses were performed in 96 well plates with 250ul of early log phase algae culture dosed with various volumes of *Beauveria bassiana* O379 (NRRL-67406) cell-free supernatant. Two wells were dosed for each volume. The assays were incubated under fluorescent lights for three days. The assays were scored by transferring 200ul from each well into a black 96 well plate then reading for fluorescence of Chlorophyll A in a microplate reader (Ex: 440nm, Em: 680nm). %Inhibitions were calculated by comparing the Relative Fluorescence Units of the treated wells to that of the untreated wells.

Dose responses on *Chara sp.* were performed by placing a sprig of *Chara* in a 15ml falcon tube containing various dilutions of *Beauveria bassiana* O379 (NRRL-67406) cell-free supernatant in mussel water. There were two replications per treatment. After five days, the tubes were scored visually.

Dose responses on *Spirogyra sp.* were conducted in 48 well plates. Strands of *Spirogyra* were transferred into 750ul of EREM medium in 48 well plates, and then the plates were dosed with various volumes of cell-free supernatant, two reps per treatment. After three days the plates are scored visually for health of the strands. Wells where all cells were dead were scored "+", wells where some cell were dead and some were alive were scored "+/-", and wells with healthy strands were scored "-".

Rice Algae Assay. *Beauveria bassiana* O379 (NRRL-67406) was tested in a bioassay meant to simulate an algae infestation in a rice field. The assay was conducted in clear plastic cups containing small pots of rice. The cups were filled with 100ml of a mixed culture of *Chlamydomonas reinhardtii, Anabaena sp., Navicula sp.,* and *Spirogyra sp.* The cups were treated with 5ml (5% v/v) *Beauveria bassiana* O379 (NRRL-67406) cell-free supernatant and incubated under lights for a total of 14 days. On days 4, 7, and 14 after treatment, the cups were scored for fluorescence of Chlorophyll A (present in all algae species) and Phycocyannin (present in cyanobacteria only). % Inhibitions were calculated by comparing the treated cups to an average of three untreated cups.

**Table 7: % Inhibition of Chlorophyll A and Phycocyannin for Rice-Algae Assay.**

| | **Day 4** | | **Day 7** | | **Day 14** | |
|---|---|---|---|---|---|---|
| | **Chl A** | **Phyc** | **Chl A** | **Phyc** | **Chl A** | **Phyc** |
| ***Beauveria bassiana* O379 (NRRL-67406)** | -92.69 | 74.23 | -15.01 | 58.69 | -17.89 | 18.92 |
| ***Beauveria bassiana* O379 (NRRL-67406)** | -21.77 | 80.29 | -34.39 | 67.70 | 12.62 | -46.90 |
| **Copper-1** | 69.99 | 86.15 | 15.02 | 96.18 | -109.23 | 90.19 |
| **Copper-2** | 53.40 | 91.23 | 21.02 | 97.31 | -8.19 | 95.25 |

*Beauveria bassiana* O379 (NRRL-67406) inhibited the growth of *Anabaena sp.* by approximately 75% on day four, and by approximately 60% on day 7. However, total chlorophyll A increased on both days. By day 14 changes in pigment concentrations were inconsistent between reps. No negative effects were seen in any of the treated plants.

A mixed algae culture was prepared by combining 1.6 liters of 1/8 Hoagland's Medium with 100ml each of mature cultures of *Chlamydomonas reinhardtii, Anabaena sp., Navicula sp.,* and *Spirogyra sp.* 100ml of the culture was added into clear plastic cups containing 5.5x5.5cm pots of two week old *Oryza sativa* grown in a clay aggregate soil. Two cups were treated with 5ml of *Beauveria bassiana O379 (NRRL-67406)*, two cups were treated with 1ppm Copper (from Pondmaster Aquatic Algaecide) and thee cups were left untreated. The cups were incubated under full spectrum lights for a total of 14 days. On days 4, 7, and 14 after treatment, the cups were scored for fluorescence of Chlorophyll A and Phycocyannin using a spectrophotometer. Three milliliter samples of the culture were pipetted into clear sided plastic cuvettes and read for fluorescence at Ex:440nm Em:680nm for Chlorophyll A, and at Ex:630nm Ex:660nm for Phycocyannin. % Inhibitions were calculated by comparing the treated cups to an average of three untreated cups.

### Example 2.

*Brevibacillus brevis* HNYM3, O989 (NRRL-B67408) was discovered as a verified hit on *Chlaymdomonas reinhardtii* and was identified by 16S rRNA as *Brevibacillus brevis* HNYM3, O989 (NRRL-B67408). Fermented supernatant of *Brevibacillus brevis* HNYM3, O989 (NRRL-B67408) was tested on a broad spectrum of algae species, and it was found to have a very broad spectrum of activity. The only tested species that it does not have activity on is the green algae *Scenedesmus quadricata.* It has greater than 75% activity on all remaining species other than *Oscillatoria tenius.* LC50s for *Brevibacillus brevis* HNYM3, O989 (NRRL-B67408) are moderately low, shown in Table 8.

When tested in a system that simulates an algae infested rice field, *Brevibacillus brevis* HNYM3, O989 (NRRL-B67408) did not perform; there was no notable change in Chlorophyll A content, and there was a notable increase in Phycocyannin content four and seven days after treatment. This suggests the *Brevibacillus brevis* HNYM3, O989 (NRRL-B67408) was promoting the growth of *Anabaena sp.* in this test, even though it has been seen to control the same species at low doses *in vitro.*

**Table 8: Brevibacillus brevis HNYM3, O989 (NRRL-B67408) Spectrum and LC50 Summary.**

| **Algae Type** | **Spectrum Species** | **Activity** | **LC 50 (%v/v)** |
|---|---|---|---|
| Green Algae | *Chlamydomonas reinhardtii* | Active | 0.5%-1% |
| | *Pesudokirchneriella subacpitata* | Active | 1.6% - 2% |
| | *Scenedesmus quadricata* | Not Active | |
| Blue-Green Algae | *Anabaena sp.* | Active | 0.5%-1% |
| | *Oscillatoria tenius* | Somewhat Active | |
| | *Microcystis aeruginosa* | Active | |
| Diatom | *Navicula sp.* | Active | |
| Macroalgae | *Chara sp.* | Active | <1% |
| | *Spirogyra sp.* | Active | 0.7% - 1.3% |

*Beauveria brevis* HNYM3, O989 (NRRL-B67408) was isolated from soil and was picked from V8 agar, and grown in V8 medium for initial screening. *Beauveria brevis* HNYM3, O989 (NRRL-B67408) was identified as an initial hit on *Chlamydomonas reinhardtii* and its activity was verified.

Initial screening was conducted by dosing 750ul of early log phase *Chamydomonas reinhardtii* culture (Modified Bold 3N Medium, pH 6.2) with 100µl of cell free supernatant in a 48 well plate. Two wells were dosed per treatment. The plates were incubated under full spectrum fluorescent lights for three days. Isolates screened on *Chlamydomonas reinhardtii* were scored visually by comparing the treated wells to the untreated wells. The isolates that resulted in wells that appeared 75% less green than the untreated wells were considered hits.

Identifying the Microbe Species Name. DNA was extracted from *Beauveria brevis* HNYM3, O989 (NRRL-B67408) using the MoBio UltraClean Microbial DNA Isolation Kit on 3/12/2012 and PCR was performed using the FD1/RD1 primers the next day. The PCR product was purified using the MoBio UltraClean PCR Clean-Up Kit and was submitted to Davis Sequencing. A consensus sequence was assembled using BioEdit and was searched in the BLAST nucleotide database and EZtaxon. The microbe was identified as *Brevibacillus brevis* HNYM3, O989 (NRRL-B67408).

Spectrum Testing. Microalgae. *Beauveria brevis* HNYM3, O989 (NRRL-B67408) was tested on a broad spectrum of algae species. *Scenedesmus quadricata* is the only species that it is not active on, and out of the remaining species *Oscillatoria tenius* is the only species it has less than 75% control of.

**Table 9: Beauveria brevis HNYM3, O989 (NRRL-B67408) Spectrum Activity on Microalgae.**

| **Algae Type** | | | **% Inhibition** | **Activity** |
|---|---|---|---|---|
| | **Spectrum Species** | **1st** | **2nd** | |
| Green Algae | *Pesudokirchneriella subacpitata* | 86.7 | 88.1 | Active |
| | *Scenedesmus quadricata* | 37.1 | 42.3 | Not Active |
| Blue-Green Algae | *Anabaena sp.* | 87.4 | 87.7 | Active |
| | *Oscillatoria tenius* | 61.8 | 58.4 | Somewhat Active |
| | *Microcystis aeruginosa* | 87.3 | 89.3 | Active |
| Diatom | *Navicula sp.* | 78.8 | 82.7 | Active |

Spectrum testing on microalgal species was carried out in a manner very similar to the initial screening on *Pseudokirchneriella subcapitata.* Testing was carried out in 48 well plates with 100ul of cell free supernatant dosed into 750µl of algae culture. Rather than an initial test followed by two verifications, two separate fermentations were concurrently tested for spectrum testing. If greater than 75% inhibition was seen for each fermentation, the isolate was considered to be "Active" on that spectrum species. If one or both of the fermentations yielded between 50% and 75% inhibition, the isolate was considered to be "Somewhat Active" on the spectrum species.

Macro Algae. *Beauveria brevis* HNYM3, O989 (NRRL-B67408) was tested on two species of macroalgae, *Spirogyra sp.* and *Chara sp.* Tests were scored visually. Thee fermentations were tested on *Chara sp.* and scores of 4 or above were considered active. One fermentation was tested *Spirogyra sp.* in four replicates. At least three of the four replicates must show strong activity to be considered a hit.

**Table 10: Beauveria brevis HNYM3, O989 (NRRL-B67408) Spectrum Activity on Chara sp.**

| | **Verification** | **Score** | **Activity** |
|---|---|---|---|
| ***Chara sp.*** | 1st | 4 | Active |
| | 2nd | 4 | Active |
| | 3rd | 5 | Active |

**Table 11: Beauveria brevis HNYM3, O989 (NRRL-B67408) Spectrum Activity on Spirogyra sp.**

| | **Score** | **Activity** |
|---|---|---|
| ***Spirogyra sp.*** | ++++ | Active |

Spectrum testing on *Chara sp.* was performed by placing a sprig of *Chara* in a 15milliliter falcon tube containing 9 milliliters of mussel water and 1milliliter of cell-free supernatant. After five days, the tubes were scored visually. Uniformly yellowed or browned sprigs were scored "2", partially yellowed or browned sprigs were scored "1", and healthy sprigs were scored "0". Three reps were tested for each sample. The three scores were added together. A score of "4" or more was considered "Active". A score of "3" was considered "Somewhat Active". Only one fermentation was tested for *Spirogyra sp.* Strands of *Spirogyra* were transferred into 750µl of EREM medium in 48 well plates, then the plates were dosed with 100ul of cell-free supernatant, four reps per treatment. After three days the plates were scored visually for health of the strands. Isolates were considered active if the strands died in three of the four replicates.

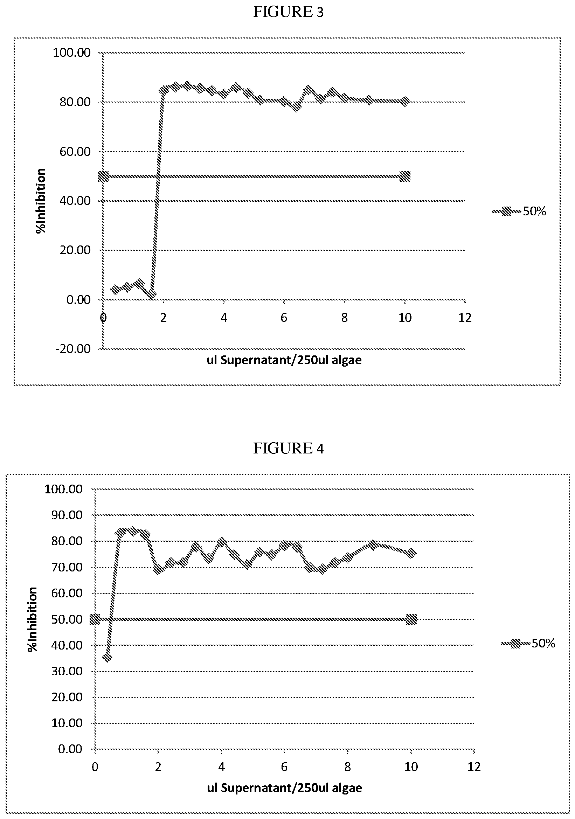

Dose Responses. Various dose responses of *Beauveria brevis* HNYM3, O989 (NRRL-B67408) cell-free supernatant were determined. Microalgae dose responses were performed using 96 well plates. (FIGURE 3 and FIGURE 4) Macroalgae dose responses were performed in either 48 well plates *(Spirogyra sp.)* or in 15ml falcon centrifuge tubes (*Chara sp.).* (Table 12 and Table 13)

**Table 12: Dose Response of Beauveria brevis HNYM3, O989 (NRRL-B67408) on Spirogyra sp. in a 48 well plate.**

| | **% *Beauveria brevis* v/v** | | | | | | | |
|---|---|---|---|---|---|---|---|---|
| | **12** | **9.1** | **6.3** | **3.2** | **1.3** | **0.7** | **0.1** | **0** |
| ***Spirogyra sp.*** | + | + | + | + | +/- | - | - | - |
| | + | + | + | + | +/- | - | - | - |

**Table 13: Dose Response of Beauveria brevis HNYM3, O989 (NRRL-B67408) on Chara sp. in 15ml tubes.**

| | ***%Beauveria** brevis* ***O379 (NRRL-67406)* Supernatant v/v** | | |
|---|---|---|---|
| | **5%** | **2.50%** | **1%** |
| ***Chara sp.*** | Active | Active | Active |

Microalgae dose responses were performed in 96 well plates with 250µl of early log phase algae culture dosed with various volumes of *Beauveria brevis* HNYM3, O989 (NRRL-B67408) cell-free supernatant. Two wells were dosed for each volume. The assays were incubated under fluorescent lights for three days. The assays were scored by transferring 200ul from each well into a black 96 well plate then reading for fluorescence of Chlorophyll A in a microplate reader (Ex: 440nm, Em: 680nm). %Inhibitions were calculated by comparing the Relative Fluorescence Units of the treated wells to that of the untreated wells.

Dose responses on *Chara sp.* were performed by placing a sprig of *Chara* in a 15ml falcon tube containing various dilutions of *Beauveria bassiana O379 (NRRL-67406)* cell-free supernatant in mussel water. There were two replications per treatment. After five days, the tubes were scored visually.

Dose responses on *Spirogyra sp.* were conducted in 48 well plates. Strands of *Spirogyra* were transferred into 750ul of EREM medium in 48 well plates, and then the plates were dosed with various volumes of cell-free supernatant, two reps per treatment. After three days the plates are scored visually for health of the strands. Wells where all cells were dead were scored "+", wells where some cell were dead and some were alive were scored "+/-", and wells with healthy strands were scored "-".

Rice Algae Assay. *Beauveria brevis* HNYM3, O989 (NRRL-B67408) was tested in a bioassay meant to simulate an algae infestation in a rice field. The assay was conducted in clear plastic cups containing small pots of rice. The cups were filled with 100ml of a mixed culture of *Chlamydomonas reinhardtii, Anabaena sp., Navicula sp.,* and *Spirogyra sp.* The cups were treated with 5ml (5% v/v) *Beauveria brevis* HNYM3, O989 (NRRL-B67408) cell-free supernatant and incubated under lights for a total of 14 days. On days 4, 7, and 14 the cups were scored for fluorescence of Chlorophyll A (present in all algae species) and Phycocyannin (present in cyanobacteria only). %Inhibitions were calculated by comparing the treated cups to an average of three untreated cups. (Table 14)

**Table 14: % Inhibition of Chlorophyll A and Phycocyannin for Rice-Algae Assay.**

| | **Day 4** | | **Day 7** | | **Day 14** | |
|---|---|---|---|---|---|---|
| | **Chl A** | **Phyc** | **Chl A** | **Phyc** | **Chl A** | **Phyc** |
| ***Beauveria brevis*** | -5.76 | -92.88 | 25.77 | -33.40 | 13.45 | 22.59 |
| ***Beauveria brevis*** | -11.80 | -55.54 | 37.09 | -30.06 | 14.17 | -3.76 |
| **Copper-1** | 69.99 | 86.15 | 15.02 | 96.18 | -109.23 | 90.19 |
| **Copper-2** | 53.40 | 91.23 | 21.02 | 97.31 | -8.19 | 95.25 |

No significant reductions in Chlorophyll A were seen for any of the three time points. On days 4 and 7 after treatment, there were increases in Phycocyannin levels for both replicates. No negative effects on the rice plants were seen in either of the replicates.

A mixed algae culture was prepared by combining 1.6L 1/8 Hoagland's Medium with 100ml each of mature cultures of *Chlamydomonas reinhardtii, Anabaena sp., Navicula sp.,* and *Spirogyra sp.* 100ml of the culture was added into clear plastic cups containing 5.5x5.5cm pots of two week old *Oryza sativa* grown in a clay aggregate soil. Two cups were treated with 5ml of O989, two cups were treated with 1ppm Copper (from Pondmaster Aquatic Algaecide) and thee cups were left untreated. The cups were incubated under full spectrum lights for a total of 14 days. On days 4, 7, and 14 after treatment, the cups were scored for fluorescence of Chlorophyll A and Phycocyannin using a spectrophotometer. Three milliliter samples of the culture were pipetted into clear sided plastic cuvettes and read for fluorescence at Ex:440nm Em:680nm for Chlorophyll A, and at Ex:630nm Ex:660nm for Phycocyannin. %Inhibitions were calculated by comparing the treated cups to an average of three untreated cups.

### Example 3.

*Streptomyces spororaveus* F18, P842 (NRRL-B67410) was discovered as a verified hit on *Chlamydomonas reinhardtii* and was identified by 16s rRNA analysis as a member of the *Streptomyces lavendulae* family with the closest species match being *Streptomyces spororaveus* F18, P842 (NRRL-B67410) and *Streptomyces norjiriensis.* It was found to have a broad spectrum of activity on representative species of microalgae and macroalgae, only lacking activity on *Scenedesmus quadricata* and *Oscillatoria tenius.* Although there is some inconsistency between fermentations, *Streptomyces spororaveus* F18, P842 (NRRL-B67410) is active at very low concentrations for some algae; a 0.05% v/v dose of P842 can yield 99% control of *Chlamydomonas reihnardtii.*

In a test meant to simulate an algae infested rice field, *Streptomyces spororaveus* F18, P842 (NRRL-B67410) had greater inhibition of Chlorophyll A than 1ppm copper seven days after treatment, though there was an increase of the blue-green algal pigment phycocyannin, suggesting that the growth of *Anabaena sp.* was promoted.

**Table 15: Streptomyces spororaveus F18, P842 (NRRL-B67410) Spectrum and LC50 Summary**

| **Algae Type** | **Spectrum Species** | **Activity** | **LC 50 (%v/v)** |
|---|---|---|---|
| Green Algae | *Chlamydomonas reinhardtii* | Active | 0.01%-0.03% |
| | *Pesudokirchneriella subacpitata* | Active | 3.6% - 4% |
| | *Scenedesmus quadricata* | Not Active | |
| Blue-Green Algae | *Anabaena sp.* | Active | 3.2% - 3.6% |
| | *Oscillatoria tenius* | Not Active | |
| | *Microcystis aeruginosa* | Active | |
| Diatom | *Navicula sp.* | Somewhat Active | |
| Macroalgae | *Chara sp.* | Active | <1% |
| | *Spirogyra sp.* | Active | 0.5% - 1% |

*Streptomyces spororaveus* F18, P842 (NRRL-B67410) was isolated from soil. The actinomycete was picked from R2A agar and fermented for screening. *Streptomyces sp. Q004 (NRRL-67409)* was identified as an initial hit on *Chlamydomonas reinhardtii* and its activity was verified.

Initial screening was conducted by dosing 750µl of early log phase *Chamydomonas reinhardtii* culture (Modified Bold 3N Medium, pH 6.2) with 100µl of cell free supernatant in a 48 well plate. Two wells were dosed per treatment. The plates were incubated under full spectrum fluorescent lights for three days. Any isolate that yielded a 75% reduction in Chlorophyll A compared to an untreated control was considered a hit. Isolates screened on *Chlamydomonas reinhardtii* were scored visually by comparing the treated wells to the untreated wells. The isolates that resulted in wells that appeared 75% less green than the untreated wells were considered hits.

Identifying the Microbe Species. DNA was extracted from *Streptomyces spororaveus* F18, P842 (NRRL-B67410) using the MoBio UltraClean Microbial DNA Isolation Kit and PCR was performed the same day using the FD1/RD1 primers. The PCR product was purified using the MoBio UltraClean PCR Clean-Up Kit and sent to Davis Sequencing. A consensus sequence was constructed in BioEdit and searched on the BLAST nucleotide database and EZtaxon.

*Streptomyces spororaveus* F18, P842 (NRRL-B67410) was identified as a member of the *Streptomyces lavendulae* family with closest matches to *Streptomyces spororaveus* F18, P842 (NRRL-B67410) and *Streptomyces norjiriensis.*

Spectrum Testing. Microalgae. *Streptomyces spororaveus* F18, P842 (NRRL-B67410) was tested on a broad spectrum of microalgal species. It has a moderately broad spectrum of activity, with strong activity on *Pseudokircherneriella* subcapitata, *Anabaena sp.,* and *Microcystis aeruginosa.* It is somewhat active on the diatom *Navicula sp.* It is not active on *Scenedesmus quadricata* or *Oscillatoria tenius.*

**Table 16: Streptomyces spororaveus F18, P842 (NRRL-B67410) spectrum Activity on Microalgae**

| **Algae Type** | **Spectrum Species** | **% Inhibition** | | |
|---|---|---|---|---|
| | | **1st** | **2nd** | **Activity** |
| Green Algae | *Pesudokirchneriella subacpitata* | 94.4 | 95.9 | Active |
| | *Scenedesmus quadricata* | 3.1 | 0.0 | Not Active |
| Blue-Green Algae | *Anabaena sp.* | 85.2 | 85.0 | Active |
| | *Oscillatoria tenius* | 0.0 | 0.0 | Not Active |
| | *Microcystis aeruginosa* | 90.2 | 90.1 | Active |
| Diato m | *Navicula sp.* | 60.7 | 71.2 | Somewhat Active |

Spectrum testing on microalgal species was carried out in a manner very similar to the initial testing on *Pseudokirchneriella subcapitata.* Testing was carried out in 48 well plates with 100ul of cell free supernatant dosed into 750µl of algae culture. Rather than an initial test followed by two verifications, two separate fermentations were concurrently tested for spectrum testing. If greater than 75% inhibition was seen for each fermentation, the isolate was considered to be "Active" on that spectrum species. If one or both of the fermentations yielded between 50% and 75% inhibition, the isolate was considered to be "Somewhat Active" on the spectrum species.

Macro Algae. *Streptomyces sp. Q004 (NRRL-67409)* was tested on two species of macroalgae, *Spirogyra sp.* and *Chara sp.* tests were scored visually. The fermentations were tested on *Chara sp.* and scores of 4 or above were considered active. One fermentation was tested *Spirogyra sp.* in four replicates. At least three of the four replicates must show strong activity to be considered a hit.

**Table 17: Streptomyces spororaveus F18, P842 (NRRL-B67410) Spectrum Activity on Chara sp.**

| | Verification | Score | Activity |
|---|---|---|---|
| *Chara sp.* | 1st | Not Tested | Not Tested |
| | 2nd | 6 | Active |
| | 3rd | 5 | Active |

**Table 18: Streptomyces spororaveus F18, P842 (NRRL-B67410) Spectrum Activity on Spirogyra sp.**

| | Score | Activity |
|---|---|---|
| *Spirogyra sp.* | ++++ | Active |

Spectrum testing on *Chara sp.* was performed by placing a sprig of *Chara* in a 15ml falcon tube containing 9ml of mussel water and 1ml of cell-free supernatant. After five days, the tubes were scored visually. Uniformly yellowed or browned sprigs were scored "2", partially yellowed or browned sprigs were scored "1", and healthy sprigs were scored "0". Three reps were tested for each sample. The three scores were added together. A score of "4" or more was considered "Active". A score of "3" was considered "Somewhat Active". Only one fermentation was tested for *Spirogyra sp.* Strands of *Spirogyra* were transferred into 750µl of EREM medium in 48 well plates, then the plates were dosed with 100µl of cell-free supernatant, four reps per treatment. After three days the plates were scored visually for health of the strands. Isolates were considered active if strands die in three of the four replicates.

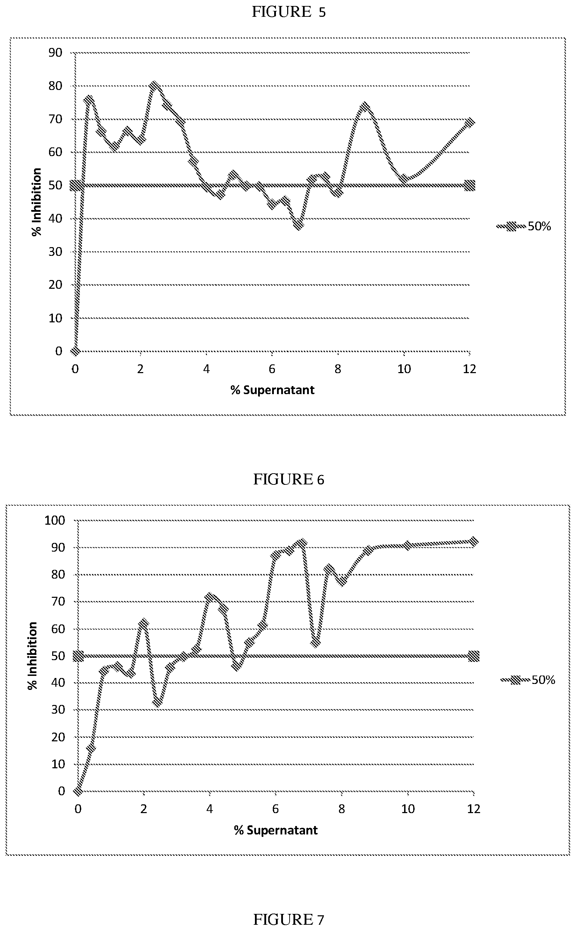

Dose Responses. Various dose responses of *Streptomyces spororaveus* F18, P842 (NRRL-B67410) cell-free supernatant were determined. Microalgae dose responses were performed using 96 well plates. (FIGURE 5 and FIGURE 6) Macroalgae dose responses were performed in either 48 well plates *(Spirogyra sp.)* or in 15ml falcon centrifuge tubes (*Chara sp.).* (Table 19 and Table 20)

**Table 19: Dose Response of Streptomyces spororaveus F18, P842 (NRRL-B67410) on Spirogyra sp. in a 48 well plate.**

| | % *Streptomyces spororaveus* F18, P842 (NRRL-B67410) v/v | | | | | | | |
|---|---|---|---|---|---|---|---|---|
| | **12** | **9.1** | **6.3** | **3.2** | **1.3** | **0.7** | **0.1** | **0** |
| *Spirogyra sp.* | + | + | + | + | + | +/- | - | - |
| | + | + | + | + | + | +/- | - | - |

**Table 20: Dose Response of Streptomyces spororaveus F18, P842 (NRRL-B67410) on Chara sp. in 15ml tubes.**

| | % *Streptomyces spororaveus* F18, P842 (NRRL-B67410) **Supernatant v/v** | | |
|---|---|---|---|
| | **5%** | **2.50%** | **1%** |
| *Chara sp.* | Active | Active | Active |

Microalgae dose responses were performed in 96 well plates with 250ul of early log phase algae culture dosed with various volumes of *Streptomyces sp. Q004 (NRRL-67409)* cell-free supernatant. Two wells were dosed for each volume. The plates were incubated under fluorescent lights for three days. The assays were scored by transferring 200µl from each well into a black 96 well plate then reading for fluorescence of Chlorophyll A in a microplate reader (Ex: 440nm, Em: 680nm). %Inhibitions were calculated by comparing the Relative Fluorescence Units of the treated wells to that of the untreated wells.

Dose responses on *Chara sp.* were performed by placing a sprig of *Chara* in a 15ml falcon tube containing various dilutions *Streptomyces spororaveus* F18, P842 (NRRL-B67410) cell-free supernatant in mussel water. There were two replications per treatment. After five days, the tubes were scored visually.

Dose responses on *Spirogyra sp.* were conducted in 48 well plates. Strands of *Spirogyra* were transferred into 750µl of EREM medium in 48 well plates, and then the plates were dosed with various volumes of cell-free supernatant, two reps per treatment. After three days the plates are scored visually for health of the strands. Wells where all cells were dead were scored "+", wells where some cell were dead and some were alive were scored "+/-", and wells with healthy strands were scored "-".

Rice Algae Assay. *Streptomyces spororaveus* F18, P842 (NRRL-B67410) was tested in a bioassay meant to simulate an algae infestation in a rice field. The assay was conducted in clear plastic cups containing small pots of rice. The cups were filled with 100ml of a mixed culture of *Chlamydomonas reinhardtii, Anabaena sp., Navicula sp.,* and *Spirogyra sp.* The cups were treated with 5ml (5% v/v) *Streptomyces sp.* Q004 (NRRL-67409) cell-free supernatant and incubated under lights for a total of 14 days. On days 4, 7, and 14 after treatment the cups were scored for fluorescence of Chlorophyll A (present in all algae species) and Phycocyannin (present in cyanobacteria only). %Inhibitions were calculated by comparing the treated cups to an average of three untreated cups.

**Table 21: % Inhibition of Chlorophyll A and Phycocyannin for Rice-Algae Assay.**

| | **Day 4** | | **Day 7** | | **Day 14** | |
|---|---|---|---|---|---|---|
| | **Chl A** | **Phyc** | **Chl A** | **Phyc** | **Chl A** | **Phyc** |
| *Streptomyces* sp. Q004 (NRRL-67409) | 39.22 | -25.22 | 64.82 | -28.87 | 42.48 | -10.45 |
| *Streptomyces* sp. Q004 (NRRL-67409) | 66.57 | 0.03 | 64.24 | -15.34 | 35.76 | -4.91 |
| Copper- 1 | 69.99 | 86.15 | 15.02 | 96.18 | -109.23 | 90.19 |
| Copper-2 | 53.40 | 91.23 | 21.02 | 97.31 | -8.19 | 95.25 |

On day 4 control of Chlorophyll A was not consistent between replicates, but there was over 50% inhibition in one of the cups. On day 7 there was consistent control of Chlorophyll A at about 64% inhibition, however, there was an increase in phycocyannin. This suggests that the content of the blue-green algal species *Anabaena sp.* had increased but the total algae content decreased. This differs greatly from copper for which there was little change in Chlorophyll A content on day 7, but there was a huge decrease in phycocyannin.

A mixed algae culture was prepared by combining 1.6L 1/8 Hoagland's Medium with 100ml each of mature cultures of *Chlamydomonas reinhardtii, Anabaena sp., Navicula sp.,* and *Spirogyra sp.* 100ml of the culture was added into clear plastic cups containing 5.5x5.5cm pots of two week old *Oryza sativa* grown in a clay aggregate soil. Two cups were treated with 5ml of *Streptomyces spororaveus* F18, P842 (NRRL-B67410) , two cups were treated with 1ppm Copper (from Pondmaster Aquatic Algaecide) and thee cups were left untreated. The cups were incubated under full spectrum lights for a total of 14 days. On days 4, 7, and 14 the cups were scored for fluorescence of Chlorophyll A and Phycocyannin using a spectrophotometer. 3ml samples of the culture were pipetted into clear sided plastic cuvettes and read for fluorescence at Ex:440nm Em:680nm for Chlorophyll A, and at Ex:630nm Ex:660nm for Phycocyannin. %Inhibitions were calculated by comparing the treated cups to an average of three untreated cups.

### Example 4.

*Paenibacillus elgii* SD17, P858 (NRRL-B67407) was discovered as a verified hit on *Chlamydomonas reinhardtii* and was identified as *Paenibacillus elgii* SD17, P858 (NRRL-B67407). The isolate was tested on a broad spectrum of algal species and was found to have a generally broad spectrum of activity. *Paenibacillus elgii* SD17, P858 (NRRL-B67407) is active on all tested species except *Scededesmus quadricata* and *Oscillatoria tenius.* While conducting spectrum testing, *Paenibacillus elgii* SD17, P858 (NRRL-B67407) was found to have less than 75% inhibition of *Pseudokirchneriella subcapitata* at a 12% v/v dose, however, in a dose response on the same species, a different fermentation of *Paenibacillus elgii* SD17, P858 (NRRL-B67407) had 95% inhibition down to a 0.8% v/v dose. This, as well as varying results on *Anabaena sp.* between fermentations, suggests that *Paenibacillus elgii* SD17, P858 (NRRL-B67407) fermentations are not very consistent, but there is potential for the supernatant to be active at very low doses.

When tested in a system designed to mimic an algae infested rice field, *Paenibacillus elgii* SD17, P858 (NRRL-B67407) performed more similarly to 1ppm Copper in terms of inhibition of Chlorophyll A and Phycocyannin than any of the other top Algaecide hits for days three and seven after treatment.

**Table 22: Paenibacillus elgii SD17, P858 (NRRL-B67407) Spectrum and LC50 Summary.**

| **Algae Type** | **Spectrum Species** | **Activity** | **LC 50 (%v/v)** |
|---|---|---|---|
| Green Algae | *Chlamydomonas reinhardtii* | Active | 0.5% - 1% |
| | *Pesudokirchneriella subacpitata* | Somewhat Active | 0.4% - 0.8% |
| | *Scenedesmus quadricata* | Not Active | |
| Blue-Green Algae | *Anabaena sp.* | Active | <0.4% |
| | *Oscillatoria tenius* | Not Active | |
| | *Microcystis aeruginosa* | Active | |
| Diatom | *Navicula sp.* | Active | |
| Macroalgae | *Chara sp.* | Active | 1%-2.5% |
| | *Spirogyra sp.* | Active | 0.5% - 1% |

*Paenibacillus elgii* SD17, P858 (NRRL-B67407) was isolated from moss and soil. *Paenibacillus elgii* SD17, P858 (NRRL-B67407) was screened on *Chlaymdomonas reinhardtii* and identified as an initial hit. Its activity was verified.

Initial screening was conducted by dosing 750µl of early log phase *Chamydomonas reinhardtii* culture (Modified Bold 3N Medium, pH 6.2) with 100µl of cell free supernatant in a 48 well plate. Two wells were dosed per treatment. The plates were incubated under full spectrum fluorescent lights for three days. Any isolate that yields a 75% reduction in Chlorophyll A compared to an untreated control was considered a hit. Isolates screened on *Chlamydomonas reinhardtii* were scored visually by comparing the treated wells to the untreated wells. The isolates that resulted in wells that appeared 75% less green than the untreated wells were considered hits.

Identifying the Microbe species name. 16S rRNA from *Paenibacillus elgii* SD17, P858 (NRRL-B67407) was amplified via Colony PCR using the FD1/RD1 primers. It was sent to Davis Sequencing and the sequences were aligned in BioEdit. The resulting consensus sequence was searched on the BLAST nucleotide database and EZtaxon. The microbe was identified as *Paenibacillus elgii* SD17, P858 (NRRL-B67407).

Spectrum Testing. Microalgae. *Paenibacillus elgii* SD17, P858 (NRRL-B67407) was tested on a broad spectrum of microalgal species. The resulting spectrum of activity is moderately broad with strong activity on *Anabaena sp., Microcystis aeruginosa,* and *Navicula sp*.; weaker activity on *Pseudokirchneriella subcapitata*; and no activity on *Scenedesmus quadricata* or *Oscillatoria tenius.*

**Table 23: Paenibacillus elgii SD17, P858 (NRRL-B67407) Spectrum Activity on Microalgae.**

| **Algae Type** | **Spectrum Species** | **% Inhibition** | | |
|---|---|---|---|---|
| | | **1st** | **2nd** | **Activity** |
| Green Algae | *Pesudokirchneriella subacpitata* | 70.9 | 76.3 | Somewhat Active |
| | *Scenedesmus quadricata* | 5.4 | 9.6 | Not Active |
| Blue-Green Algae | *Anabaena sp.* | 94.7 | 95.0 | Active |
| | *Oscillatoria tenius* | 30.4 | 16.7 | Not Active |
| | *Microcystis aeruginosa* | 82.5 | 83.5 | Active |
| Diatom | *Navicula sp.* | 88.6 | 92.7 | Active |

Spectrum testing on microalgal species was carried out in a manner very similar to the initial screening on *Pseudokirchneriella subcapitata.* Testing was carried out in 48 well plates with 100ul of cell free supernatant dosed into 750µl of algae culture. Rather than an initial test followed by two verifications, two separate fermentations were concurrently tested for spectrum testing. If greater than 75% inhibition was seen for each fermentation, the isolate was considered to be "Active" on that spectrum species. If one or both of the fermentations yielded between 50% and 75% inhibition, the isolate was considered to be "Somewhat Active" on the spectrum species.

Macro Algae. *Paenibacillus elgii* SD17, P858 (NRRL-B67407) was tested on two species of macroalgae, *Spirogyra sp.* and *Chara sp.* Tests were scored visually. One fermentation was tested on *Chara sp.* and scores of 4 or above were considered active. One fermentation was tested *Spirogyra sp.* in four replicates. At least three of the four replicates must show strong activity to be considered a hit.

**Table 24: Paenibacillus elgii SD17, P858 (NRRL-B67407) Spectrum Activity on Chara sp.**

| | **Verification** | **Score** | **Activity** |
|---|---|---|---|
| *Chara sp.* | 1 st | 4 | Active |
| | 2nd | Not tested | Not tested |

**Table 25: Paenibacillus elgii SD17, P858 (NRRL-B67407) Spectrum Activity on Spirogyra sp.**

| | Score | Activity |
|---|---|---|
| *Spirogyra sp.* | ++++ | Active |

Spectrum testing on *Chara sp.* was performed by placing a sprig of *Chara* in a 15ml falcon tube containing 9ml of mussel water and 1ml of cell-free supernatant. After five days, the tubes were scored visually. Uniformly yellowed or browned sprigs were scored "2", partially yellowed or browned sprigs were scored "1", and healthy sprigs were scored "0". Three reps were tested for each sample. The three scores were added together. A score of "4" or more was considered "Active". A score of "3" was considered "Somewhat Active". Only one fermentation was tested for *Spirogyra sp.* Strands of *Spirogyra* were transferred into 750ul of EREM medium in 48 well plates, then the plates were dosed with 100µl of cell-free supernatant, four reps per treatment. After three days the plates are scored visually for health of the strands. Isolates are considered active if strands die in three of the four replicates.

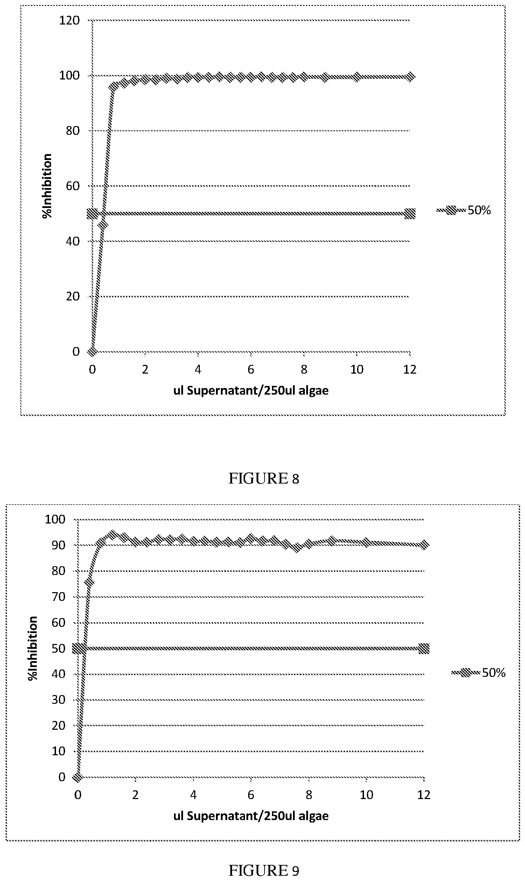

Dose Responses. Various dose responses of *Paenibacillus elgii* SD17, P858 (NRRL-B67407) cell-free supernatant were determined. Microalgae dose responses were performed using 96 well plates. (FIGURE 7 and FIGURE 8) Macroalgae dose responses were performed in either 48 well plates *(Spirogyra sp.)* or in 15ml falcon centrifuge tubes (*Chara sp.).* (Table 26 and Table 27).

Note that during spectrum testing, *Paenibacillus elgii* SD17, P858 (NRRL-B67407) was determined to only be "Somewhat Active" at a 12%v/v dose, but in this test, it yielded 95% inhibition down to a 0.8% dose. This suggests that *Paenibacillus elgii* SD17, P858 (NRRL-B67407)'s activity varies notably between fermentations.

**Table 26: Dose Response of Paenibacillus elgii SD17, P858 (NRRL-B67407) on Spirogyra sp. in a 48 well plate.**

| | **% P858 v/v** | | | | | | | |
|---|---|---|---|---|---|---|---|---|
| | **12** | **9.1** | **6.3** | **3.2** | **1.3** | **0.7** | **0.1** | **0** |
| *Spirogyra sp.* | + | + | +/- | + | + | +/- | - | - |
| | + | + | + | + | +/- | + | - | - |

**Table 27: Dose Response of Paenibacillus elgii SD17, P858 (NRRL-B67407) on Chara sp. in 15ml tubes.**

| | **% *Paenibacillus elgii* SD17, P858 (NRRL-B67407) Supernatant v/v** | | |
|---|---|---|---|
| | **5%** | **2.50%** | **1%** |
| *Chara sp.* | Active | Active | Not Active |

Microalgae dose responses were performed in 96 well plates with 250µl of early log phase algae culture dosed with various volumes of *Paenibacillus elgii* SD17, P858 (NRRL-B67407) cell-free supernatant. Two wells were dosed for each volume. The plates were incubated under fluorescent lights for three days. The assays were scored by transferring 200µl from each well into a black 96 well plate then reading for fluorescence of Chlorophyll A in a microplate reader (Ex: 440nm, Em: 680nm). %Inhibitions were calculated by comparing the Relative Fluorescence Units of the treated wells to that of the untreated wells.

Dose responses on *Chara sp.* were performed by placing a sprig of *Chara* in a 15ml falcon tube containing various dilutions of P858 cell-free supernatant in mussel water. There were two replications per treatment. After five days, the tubes were scored visually.

Dose responses on *Spirogyra sp.* were conducted in 48 well plates. Strands of *Spirogyra* were transferred into 750µl of EREM medium in 48 well plates, and then the plates were dosed with various volumes of cell-free supernatant, two reps per treatment. After three days the plates were scored visually for health of the strands. Wells where all cells were dead were scored "+", wells where some cell were dead and some were alive were scored "+/-", and wells with healthy strands were scored "-".

Rice Algae Assay. *Paenibacillus elgii* SD17, P858 (NRRL-B67407) was tested in a bioassay meant to simulate an algae infestation in a rice field. The assay was conducted in clear plastic cups containing small pots of rice. The cups were filled with 100ml of a mixed culture of *Chlamydomonas reinhardtii, Anabaena sp., Navicula sp.,* and *Spirogyra sp.* The cups were treated with 5ml (5% v/v) *Paenibacillus elgii* SD17, P858 (NRRL-B67407) cell-free supernatant and incubated under lights for a total of 14 days. On days 4, 7, and 14 after treatment the cups were scored for fluorescence of Chlorophyll A (present in all algae species) and Phycocyannin (present in cyanobacteria only). %Inhibitions were calculated by comparing the treated cups to an average of three untreated cups.

**Table 28: % Inhibition of Chlorophyll A and Phycocyannin for Rice-Algae Assay.**

| | Day 4 | | Day 7 | | Day 14 | |
|---|---|---|---|---|---|---|
| | Chl A | Phyc | Chl A | Phyc | Chl A | Phyc |
| *Paenibacillus elgii* SD17, P858 (NRRL-B67407) | 63.87 | 74.37 | 15.81 | 61.43 | 23.11 | 1.96 |
| *Paenibacillus elgii* SD17, P858 (NRRL- | 67.58 | 82.77 | -28.83 | 60.10 | -45.46 | 32.17 |
| B67407) | | | | | | |
| Copper-1 | 69.99 | 86.15 | 15.02 | 96.18 | -109.23 | 90.19 |
| Copper-2 | 53.40 | 91.23 | 21.02 | 97.31 | -8.19 | 95.25 |

The activity of *Paenibacillus elgii* SD17, P858 (NRRL-B67407) in this assay was similar to the activity or 1ppm Copper. Four days after treatment both had a greater than 50% inhibition of Chlorophyll A, and an approximately greater than 75% inhibition of Phycocyannin. On day seven there was no control of Chlorophyll A for either algaecide, but continued control of Phycocyannin. However, *Paenibacillus elgii* SD17, P858 (NRRL-B67407) yielded only a 60% control of the cyanobacterial pigment, while copper had greater than 95% control. Copper continued to control Phycocyannin on day 14, while control from P858 ceased before then.

A mixed algae culture was prepared by combining 1.6L 1/8 Hoagland's Medium with 100ml each of mature cultures of *Chlamydomonas reinhardtii, Anabaena sp., Navicula sp.,* and *Spirogyra sp.* 100ml of the culture was added into clear plastic cups containing 5.5x5.5cm pots of two week old *Oryza sativa* grown in a clay aggregate soil. Two cups were treated with 5ml of *Paenibacillus elgii* SD17, P858 (NRRL-B67407), two cups were treated with 1ppm Copper (from Pondmaster Aquatic Algaecide) and thee cups were left untreated. The cups were incubated under full spectrum lights for a total of 14 days. On days 4, 7, and 14 after treatment the cups were scored for fluorescence of Chlorophyll A and Phycocyannin using a spectrophotometer. Thee milliliter samples of the culture were pipetted into clear sided plastic cuvettes and read for fluorescence at Ex:440nm Em:680nm for Chlorophyll A, and at Ex:630nm Ex:660nm for Phycocyannin. %Inhibitions were calculated by comparing the treated cups to an average of three untreated cups.

### Example 5.

*Streptomyces* sp. Q004 (NRRL-67409) was identified as a verified hit on *Chlamydomonas reinhardtii* and was identified by 16S rRNA sequencing as a member of the *Streptomyces lavendulae* group, likely *Streptomyces spororaveus* or *Streptomyces nojiriensis. Streptomyces* sp. Q004 (NRRL-67409) was tested on a broad spectrum of algae species, and it was found to have a somewhat broad spectrum of activity. Streptomyces sp. Q004 (NRRL-67409) is active on all tested species except for *Scenedesmus quadricata, Anabaena* sp., and *Oscillatoria tenius.* While this gives the isolate a somewhat broad spectrum compared to most algaecide verified hits, it has the narrowest spectrum of the top five algaecide hits. Additionally, Streptomyces sp. Q004 (NRRL-67409) has comparatively high LC50s for most algae species.

In a test meant to simulate an algae infested rice field, *Streptomyces* sp. Q004 (NRRL-67409) demonstrated some inhibition of Chlorophyll A on days three and seven, though this activity was not consistent between replicates. *Streptomyces* sp. Q004 (NRRL-67409) did consistently boost the concentration of the cyanobacterial pigment phycocyannin in this test for days three and seven.

**Table 29: Streptomyces sp. Q004 (NRRL-67409) Spectrum and LC50 Summary**

| **Algae Type** | **Spectrum Species** | **Activity** | **LC 50 (%v/v)** |
|---|---|---|---|
| Green Algae | *Chlamydomonas reinhardtii* | Active | 0.5%-1% |
| | *Pesudokirchneriella subacpitata* | Active | 10%-12% |
| | *Scenedesmus quadricata* | Not Active | |
| Blue-Green Algae | *Anabaena sp.* | Not Active | 8.0% - 8.8% |
| | *Oscillatoria tenius* | Not Active | |
| | *Microcystis aeruginosa* | Active | |
| Diatom | *Navicula sp.* | Somewhat Active | |
| Macroalgae | *Chara sp.* | Active | 5%-10% |
| | *Spirogyra sp.* | Active | >5% |

*Streptomyces sp. Q004 (NRRL-67409)* was identified as an initial hit on *Chlamydomonas reinhardtii* and its activity was verified.

Initial screening was conducted by dosing 750µl of early log phase *Chamydomonas reinhardtii* culture (Modified Bold 3N Medium, pH 6.2) with 100µl of cell free supernatant in a 48 well plate. Two wells were dosed per treatment. The plates were incubated under full spectrum fluorescent lights for three days. Any isolate that yields a 75% reduction in Chlorophyll A compared to an untreated control was considered a hit. Isolates screened on *Chlamydomonas reinhardtii* were scored visually by comparing the treated wells to the untreated wells. The isolates that resulted in wells that appeared 75% less green than the untreated wells were considered hits.

Identifying the Microbe species. DNA was extracted from *Streptomyces* sp. Q004 (NRRL-67409) using the MoBio UltraClean Microbial DNA Isolation Kit and PCR was performed on the same day using the FD1 and RD1 primers. The PCR reaction was purified using the MoBio UltraClean PCR Clean-Up Kit and sent to Davis Sequencing. The sequences were aligned in BioEdit and a consensus sequence was generated. The consensus sequence was searched in the BLAST nucleotide database and EZtaxon.

Streptomyces sp. Q004 (NRRL-67409) was identified as a member of the *Streptomyces lavendulae* group with the closest matches being *Streptomyces spororaveus* and *Streptomyces nojiriensis.*

Spectrum Testing. Microalgae. *Streptomyces* sp. Q004 (NRRL-67409) was tested on a broad spectrum of microalgal species. Out of the top five algaecide hits, *Streptomyces* sp. Q004 (NRRL-67409) has the narrowest spectrum, with no activity on *Scenedesmus quadricata, Anabaena sp.,* or *Oscillatoria tenius,* and less than 75% inhibition of *Navicula sp.*

**Table 30: Streptomyces sp. Q004 (NRRL-67409) Spectrum Activity on Microalgae**

| **Algae Type** | **Spectrum Species** | **% Inhibition** | | **Activity** |
|---|---|---|---|---|
| | | **1st** | **2nd** | |
| Green Algae | *Pesudokirchneriella subacpitata* | 80.9 | 84.1 | Active |
| | *Scenedesmus quadricata* | 38.3 | 25.9 | Not Active |
| Blue-Green Algae | *Anabaena sp.* | 33.9 | 56.1 | Not Active |
| | *Oscillatoria tenius* | 20.51 | 15.33 | Not Active |
| | *Microcystis aeruginosa* | 93.1 | 92.2 | Active |
| Diatom | *Navicula sp.* | 53.3 | 71.6 | Somewhat Active |

Spectrum testing on microalgal species was carried out in a manner very similar to the initial testing on *Pseudokirchneriella subcapitata.* Testing was carried out in 48 well plates with 100ul of cell free supernatant dosed into 750µl of algae culture. Rather than an initial test followed by two verifications, two separate fermentations were concurrently tested for spectrum testing. If greater than 75% inhibition was seen for each fermentation, the isolate was considered to be "Active" on that spectrum species. If one or both of the fermentations yielded between 50% and 75% inhibition, the isolate was considered to be "Somewhat Active" on the spectrum species.

Macro Algae. *Streptomyces* sp. Q004 (NRRL-67409) was tested on two species of macroalgae, *Spirogyra sp.* and *Chara sp.* tests were scored visually. Two fermentations were tested on *Chara sp.* and scores of 4 or above were considered active. One fermentation was tested *Spirogyra sp.* in four replicates. At least three of the four replicates must show strong activity to be considered a hit.

**Table 31: Streptomyces sp. Q004 (NRRL-67409) Spectrum Activity on Chara sp.**

| | **Verification** | **Score** | **Activity** |
|---|---|---|---|
| *Chara sp.* | 1st | Not Tested | |
| | 2nd | 6 | Active |
| | 3rd | 4 | Active |

**Table 32: Streptomyces sp. Q004 (NRRL-67409) Spectrum Activity on Spirogyra sp.**

| | **Score** | **Activity** |
|---|---|---|
| *Spirogyra sp.* | ++++ | Active |

Spectrum testing on *Chara sp.* was performed by placing a sprig of *Chara* in a 15ml falcon tube containing 9mls of mussel water and 1ml of cell-free supernatant. After five days, the tubes were scored visually. Uniformly yellowed or browned sprigs were scored "2", partially yellowed or browned sprigs were scored "1", and healthy sprigs were scored "0". Three reps were tested for each sample. The three scores were added together. A score of "4" or more was considered "Active". A score of "3" was considered "Somewhat Active". Only one fermentation was tested for *Spirogyra sp.* Strands of *Spirogyra* were transferred into 750µl of EREM medium in 48 well plates, then the plates were dosed with 100µl of cell-free supernatant, four reps per treatment. After three days the plates are scored visually for health of the strands. Isolates are considered active if strands die in three of the four replicates.

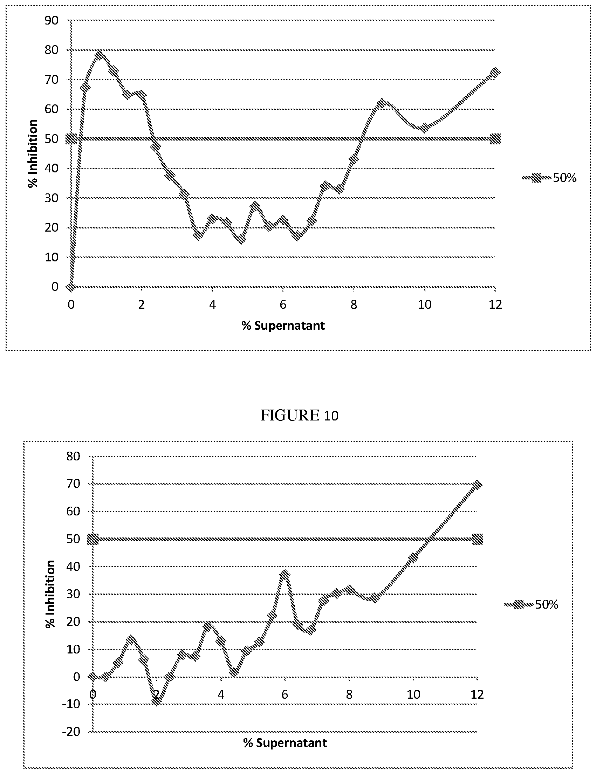

Dose Responses. Various dose responses of *Streptomyces* sp. Q004 (NRRL-67409) cell-free supernatant were determined. Microalgae dose responses were performed using 96 well plates. (FIGURE 9 and FIGURE 10) Macroalgae dose responses were performed in either 48 well plates *(Spirogyra sp.)* or in 15ml falcon centrifuge tubes (*Chara sp.).* (Table 33 and Table 34)

**Table 33: Dose Response of Streptomyces sp. Q004 (NRRL-67409) on Spirogyra sp. in a 48 well plate.**

| | **% *Streptomyces* sp. Q004 (NRRL-67409) v/v** | | | | | | | |
|---|---|---|---|---|---|---|---|---|
| | **12** | **9.1** | **6.3** | **3.2** | **1.3** | **0.7** | **0.1** | **0** |
| *Spirogyra sp.* | +/- | +/- | +/- | +/- | +/- | - | - | - |
| | + | +/- | +/- | +/- | +/- | - | - | - |

**Table 34: Dose Response of Streptomyces sp. Q004 (NRRL-67409) on Chara sp. in 15ml tubes.**

| | **% *Streptomyces* sp. Q004 (NRRL-67409) Supernatant v/v** | | |
|---|---|---|---|
| | **5%** | **2.50%** | **1%** |
| *Chara sp.* | Not Active | Not Active | Not Active |

Microalgae dose responses were performed in 96 well plates with 250µl of early log phase algae culture dosed with various volumes of *Streptomyces* sp. Q004 (NRRL-67409) cell-free supernatant. Two wells were dosed for each volume. The plates were incubated under fluorescent lights for three days. The assays were scored by transferring 200µl from each well into a black 96 well plate then reading for fluorescence of Chlorophyll A in a microplate reader (Ex: 440nm, Em: 680nm). %Inhibitions were calculated by comparing the Relative Fluorescence Units of the treated wells to that of the untreated wells.

Dose responses on *Chara sp.* were performed by placing a sprig of *Chara* in a 15ml falcon tube containing various dilutions of *Streptomyces* sp. Q004 (NRRL-67409) cell-free supernatant in mussel water. There were two replications per treatment. After five days, the tubes were scored visually.

Dose responses on *Spirogyra sp.* were conducted in 48 well plates. Strands of *Spirogyra* were transferred into 750µl of EREM medium in 48 well plates, and then the plates were dosed with various volumes of cell-free supernatant, two reps per treatment. After three days the plates are scored visually for health of the strands. Wells where all cells were dead were scored "+", wells where some cell were dead and some were alive were scored "+/-", and wells with healthy strands were scored "-".

Rice Algae Assay. *Streptomyces* sp. Q004 (NRRL-67409) was tested in a bioassay meant to simulate an algae infestation in a rice field. The assay was conducted in clear plastic cups containing small pots of rice. The cups were filled with 100ml of a mixed culture of *Chlamydomonas reinhardtii, Anabaena sp., Navicula sp.,* and *Spirogyra sp.* The cups were treated with 5ml (5% v/v) *Streptomyces* sp. Q004 (NRRL-67409) cell-free supernatant and incubated under lights for a total of 14 days. On days 4, 7, and 14 after treatment the cups were scored for fluorescence of Chlorophyll A (present in all algae species) and Phycocyannin (present in cyanobacteria only). %Inhibitions were calculated by comparing the treated cups to an average of three untreated cups.

**Table 35: % Inhibition of Chlorophyll A and Phycocyannin for Rice-Algae Assay.**

| | **Day 4** | | **Day 7** | | **Day 14** | |
|---|---|---|---|---|---|---|
| | **Chl A** | **Phyc** | **Chl A** | **Phyc** | **Chl A** | **Phyc** |
| *Streptomyces* sp. Q004 (NRRL-67409) | 64.00 | -49.89 | 74.05 | -24.20 | 48.34 | 3.83 |
| *Streptomyces* sp. Q004 (NRRL-67409) | 17.73 | -33.72 | 49.31 | -31.66 | 26.82 | -18.98 |
| Copper-1 | 69.99 | 86.15 | 15.02 | 96.18 | -109.23 | 90.19 |
| Copper-2 | 53.40 | 91.23 | 21.02 | 97.31 | -8.19 | 95.25 |

*Streptomyces* sp. Q004 (NRRL-67409) had some control of Chlorophyll A on days 7 and 4 after treatment, but this was not consistent between replicates. There was an increase in Phycocyannin for both replicates on days 7 and 4, suggesting that *Streptomyces* sp. Q004 (NRRL-67409) stimulated the growth of *Anabaena sp.*

A mixed algae culture was prepared by combining 1.6L 1/8 Hoagland's Medium with 100ml each of mature cultures of *Chlamydomonas reinhardtii, Anabaena sp., Navicula sp.,* and *Spirogyra sp.* 100ml of the culture was added into clear plastic cups containing 5.5x5.5cm pots of two week old *Oryza sativa* grown in a clay aggregate soil. Two cups were treated with 5ml of *Streptomyces* sp. Q004 (NRRL-67409), two cups were treated with 1ppm Copper (from Pondmaster Aquatic Algaecide) and thee cups were left untreated. The cups were incubated under full spectrum lights for a total of 14 days. On days 4, 7, and 14 after treatment the cups were scored for fluorescence of Chlorophyll A and Phycocyannin using a spectrophotometer. 3ml samples of the culture were pipetted into clear sided plastic cuvettes and read for fluorescence at Ex:440nm Em:680nm for Chlorophyll A, and at Ex:630nm Ex:660nm for Phycocyannin. %Inhibitions were calculated by comparing the treated cups to an average of three untreated cups.

### Example Summary

Examples 1-5 are summarized in the below table.

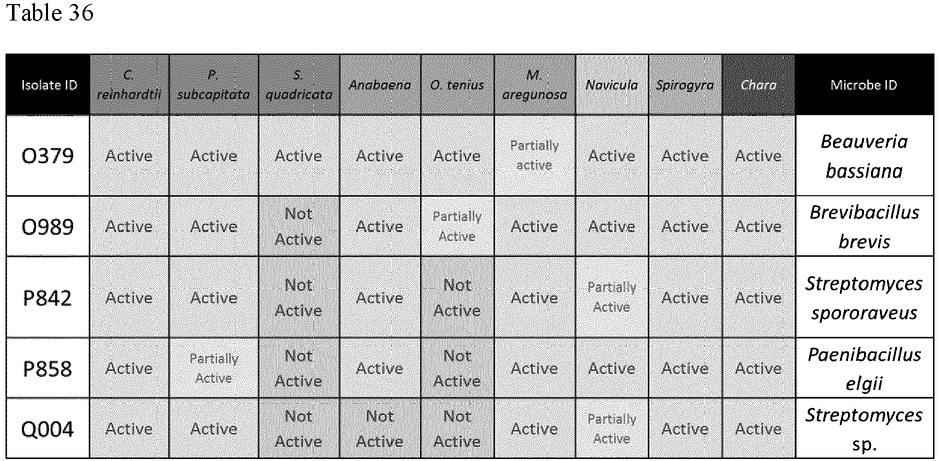

### DEPOSIT OF BIOLOGICAL MATERIAL

The following biological material has been deposited under the terms of the Budapest Treaty with the Agricultural Research Culture Collection (NRRL), 1815 N. University Street, Peoria, Illinois 61604, USA, and given the following number:

| Deposit | Accession Number | Date of Deposit |
|---|---|---|
| *Beauveria bassiana* O379 | NRRL 67406 | March 30, 2017 |
| *Paenibacillus elgii* SD17, P858 | NRRL B-67407 | March 30, 2017 |
| *Brevibacillus brevis* HNYM3, O989 | NRRL B-67408 | March 30, 2017 |
| *Streptomyces* sp. Q004 | NRRL B-67409 | March 30, 2017 |
| *Streptomyces spororaveus* F18, P842 | NRRL B-67410 | March 30, 2017 |

The strains have been deposited under conditions that assure that access to the culture will be available during the pendency of this patent application and

## Claims

1. An algicidal composition comprising: a whole cell broth, filtrate, supernatant, or extract collected from *Beauveria bassiana* 0379 (NRRL- 67406), *Brevibacillus brevis* HNYM3, 0989 (NRRL-B67408), *Streptomyces spororaveus* F18, P842 (NRRL-B67410), *Paenibacillus elgii* SD17, P858 (NRRL-B67407), or *Streptomyces* sp. Q004 (NRRL-67409) fermentation.

2. The composition of claim 1, further comprises a carrier, diluent, adjuvant or surfactant.

3. The composition of claim 1, wherein said algae comprises Chlamydomonas reinhardtii, Pesudokirchneriella subacpitata, Scenedesmus quadricata, Anabaena sp., Oscillatoria tenuis, Microcystis aeruginosa, Navicula sp., Spirogyra sp., or Char a sp.

4. The composition of claim 1, wherein said composition is in a dustable powders, soluble powders, water soluble granules, water dispersible granules, wettable powders, granules, soluble concentrates, oil miscible liquids, ultra-low volume liquids, emulsifiable concentrates, dispersible concentrates, emulsions, micro-emulsions, suspension concentrates, oil-based suspension concentrate, aerosols, fogging/smoke, capsule suspensions or seed treatment formulation.

5. The composition of claim 1, further comprising a chemical or biological algaecide.

6. The composition of claim 5, wherein said biological algaecide comprises Bacillus firmus , Bacillus thuringiensis, Bacillus pumilus, Bacillus licheniformis, Bacillus amyloliquefaciens, Bacillus subtilis, Paecilomyces lilacinus, Pasteuria penetrans, Pseudomonas sp., Brevabacillus sp., Lecanicillium sp., Ampelomyces sp., Pseudozyma sp., Streptomyces bikiniensis, Streptomyces costaricanus, Streptomyces avermitilis, Burkholderia sp., Trichoderma sp., Gliocladium sp., avermectin, Myrothecium sp., Paecilomyces spp., Sphingobacterium sp., Arthrobotrys sp., Chlorosplrnium, Neobulgaria, Daldinia, Aspergillus, Chaetomium, Lysobacter spp, Lachnum papyraceum, Verticillium suchlasporium, Arthrobotrys oligospora, Verticillium chlamydosporium, Hirsutella rhossiliensis, Pochonia chlamydosporia, Pleurotus ostreatus, Omphalotus olearius, Lampteromyces japonicas, Brevudimonas sp., or Muscodor sp.

7. The composition of claim 5, wherein said chemical algaecide comprises 3-(4-chloro-2,6-dimethylphenyl)-4-hydroxy-8-oxa-l-azaspiro[4,5]dec-3-en-2-one; 3-(4'-chloro-2,4- dimethyl[l,l'-biphenyl]-3-yl)-4-hydroxy-8-oxa-l-azaspiro- [4,5]dec-3-en-2-one; 4-[[(6-chloro- 3-pyridinyl)methyl]methylamino]-2(5H)-furanone; 4-[[(6-chloro-3- pyridinyl)methyl]cyclopropylamino]-2(5H)-furanone; 3-chloro-N2-[(IS)-I-methyl-2-(methylsulf ony l)ethyl] -N 1 - [2 -methy 1-4- [ 1 ,2 ,2 ,2 - tetrafluoro- 1 - (trifluoromethyl) ethyl] phenyl] - 1 ,2-benzenedicarboxamide; 2-cyano-N-ethyl-4-fluoro-3-methoxy-benenesulfonamide; 2-cyano- N-ethyl-3-methoxy-benzenesulfonamide; 2-cyano-3-difluoromethoxy-N-ethyl-4-fluoro- benzenesulfonamide; 2-cyano-3-fluoromethoxy-N-ethyl-benzenesulfonamide; 2-cyano-6-fluoro- 3-methoxy-N,N-dimethyl-benzenesulfonamide; 2-cyano-N-ethyl-6-fluoro-3-methoxy-N-methyl- benzenesulfonamide; 2-cyano-3-difluoromethoxy-N,N-dimethylbenzenesulfon-amide; 3- (difluoromethyl)-N-[2-(3,3-dimethylbutyl)phenyl]-I -methyl- lH-pyrazole-4- carboxamide; N- ethyl-2,2-dimethylpropionamide-2-(2,6-dichloro-D,D,D,-trifluoro-p-tolyl) hydrazone; N-ethyl- 2,2-dichloro-l-methylcyclopropane-carboxamide-2-(2,6-dichloro-D,D,D,-trifluoro-p-tolyl) hydrazone nicotine; 0-{ (E-)-[2-(4-chloro-phenyl)-2-cyano-I-(2-trifluoromethylphenyl)-vinyl] }S-methyl thiocarbonate; (E)-Nl-[(2-chloro-l,3-thiazol-5-ylmethyl)]-N2-cyano-Nl-methylacetamidine; l-(6-chloropyridin-3-ylmethyl)-7-methyl-8-nitro-l,2,3,5,6,7-hexahydro-imidazo[I,2-a]pyridin-5-ol; 4-[4-chlorophenyl-(2-butylidine-hydrazono)methyl)]phenyl mesylate; or N-Ethyl-2,2-dichloro-l-methylcyclopropanecarboxamide-2-(2,6-dichloro-trifluoro-p-tolyl)hydrazone.

8. A method for inhibiting the growth of an algae comprising the steps of:
introducing the composition of claim 1 in a location where inhibition is desired, in amounts effective for inhibiting said algae.

9. The method of claim 8, wherein said location is a liquid comprising a body of water or paint.

10. The method of claim 8, wherein said location is a solid surface selected from the group consisting of plastic, concrete, wood, fiberglass, pipes made of iron and polyvinyl chloride, and surfaces covered with coating materials and/or paint.

11. The method of claim 8, wherein said algae comprises Chlamydomonas reinhardtii, Pesudokirchneriella subacpitata, Scenedesmus quadricata, Anabaena sp., Oscillatoria tenuis, Microcystis aeruginosa, Navicula sp., Spirogyra sp., or Char a sp.

12. A method for inhibiting the growth of an algae comprising the steps of:
introducing the composition of claim 5 in a location where inhibition is desired, in amounts effective for inhibiting said algae.

13. The method of claim 12, wherein said location is a liquid comprising a body of water or paint.

14. The method of claim 12, wherein said location is a solid surface selected from the group consisting of plastic, concrete, wood, fiberglass, pipes made of iron and polyvinyl chloride, and surfaces covered with coating materials and/or paint.

15. The method of claim 12, wherein said algae comprises Chlamydomonas reinhardtii, Pesudokirchneriella subacpitata, Scenedesmus quadricata, Anabaena sp., Oscillatoria tenuis, Microcystis aeruginosa, Navicula sp., Spirogyra sp., or Char a sp.

## Patentansprüche

1. Algizide Zusammensetzung, umfassend: eine Vollzellbrühe, ein Filtrat, einen Überstand oder einen Extrakt, gesammelt aus *Beauveria bassiana* 0379 (NRRL- 67406), *Brevibacillus brevis* HNYM3, 0989 (NRRL-B67408), *Streptomyces spororaveus* F18, P842 (NRRL-B67410), *Paenibacillus elgii* SD17, P858 (NRRL- B67407) oder *Streptomyces* sp. Q004 (NRRL-67409) fermentiert.

2. Zusammensetzung nach Anspruch 1, ferner umfassend einen Träger, ein Verdünnungsmittel, ein Hilfsmittel oder ein Tensid.

3. Zusammensetzung nach Anspruch 1, wobei die Alge Chlamydomonas reinhardtii, Pesudoklrchneriella subacpitata, Scenedesmus quadricata, Anabaena sp., Oscillatoria tenuis, Microcystis aeruginosa, Navicula sp., Spirogyra sp. oder Char a sp. umfasst.

4. Zusammensetzung nach Anspruch 1, wobei die Zusammensetzung in Form von staubfähigen Pulvern, löslichen Pulvern, wasserlöslichen Granulaten, in Wasser dispergierbaren Granulaten, benetzbaren Pulvern, Granulaten, löslichen Konzentraten, ölmischbaren Flüssigkeiten, ultraniedrigvolumigen Flüssigkeiten, emulgierbaren Konzentraten, dispergierbaren Konzentraten, Emulsionen, Mikroemulsionen, Suspensionskonzentraten, Suspensionskonzentraten auf Ölbasis, Aerosolen, Vernebelung/Rauch, Kapselsuspensionen oder Saatgutbehandlungsformulierungen vorliegt.

5. Zusammensetzung nach Anspruch 1, ferner umfassend ein chemisches oder biologisches Algizid.

6. Zusammensetzung nach Anspruch 5, wobei das biologische Algizid Bacillus firmus, Bacillus thuringiensis, Bacillus pumilus, Bacillus licheniformis, Bacillus amyloliquefaciens, Bacillus subtilis, Paecilomyces lilacinus, Pasteuria penetrans, Pseudomonas sp., Brevabacillus sp., Lecanicillium sp., Ampelomyces sp., Pseudozyma sp., Streptomyces bikiniensis, Streptomyces costaricanus, Streptomyces avermitilis, Burkholderia sp., Trichoderma sp., Gliocladium sp., Avermectin, Myrothecium sp., Paecilomyces spp., Sphingobacterium sp., Arthrobotrys sp., Chlorosplrnium, Neobulgaria, Daldinia, Aspergillus, Chaetomium, Lysobacter spp, Lachnum papyraceum, Verticillium suchlasporium, Arthrobotrys oligospora, Verticillium chlamydosporium, Hirsutella rhossiliensis, Pochonia chlamydosporia, Pleurotus ostreatus, Omphalotus olearius, Lampteromyces japonicas, Brevudimonas sp. oder Muscodor sp. umfasst.

7. Zusammensetzung nach Anspruch 5, wobei das chemische Algizid 3-(4-Chlor-2,6-dimethylphenyl)-4-hydroxy-8-oxa-l-azaspiro[4,5]dec-3-en-2-on; 3-(4'-Chlor-2,4-dimethyl[l,l'-biphenyl]-3-yl)-4-hydroxy-8-oxa-l-azaspiro-[4,5]dec-3-en-2-on; 4-[[(6-Chlor-3-pyridinyl)methyl]methylamino]-2(5H)-furanon; 4-[[(6-Chlor-3-pyridinyl)methyl]cyclopropylamino]-2(5H)-furanon; 3-Chlor-N2-[(IS)-l-Methyl-2-(methylsulf ony l)ethyl]-N1-[2-Methyl-1-4- [ 1 ,2 ,2 ,2 - Tetrafluor-1-(trifluormethyl)ethyl]phenyl]-1,2-benzoldicarboxamid; 2-Cyano-N-ethyl-4-fluor-3-methoxy-benensulfonamid; 2-Cyano-N-ethyl-3-methoxy-benzolsulfonamid; 2-Cyano-3-difluormethoxy-N-ethyl-4-fluor-benzolsulfonamid; 2-Cyano-3-fluormethoxy-N-ethyl-benzolsulfonamid; 2-Cyano-6-fluor-3-methoxy-N,N-dimethyl-benzolsulfonamid; 2-Cyano-N-ethyl-6-fluor-3-methoxy-N-methyl-benzolsulfonamid; 2-Cyano-3-difluormethoxy-N,N-dimethyl-benzolsulfon-amide; 3-(Difluormethyl)-N-[2-(3,3-dimethylbutyl)phenyl]-l-methyl-IH-pyrazol-4- carboxamid; N-ethyl-2,2-dimethylpropionamid-2-(2,6-dichlor-D,D,D,-trifluor-p-tolyl) hydrazon; N-ethyl- 2,2-dichlor-l-methylcyclopropan-carboxamide-2-(2,6-dichlor-D,D,D,-trifluor-p-tolyl) hydrazon nikotin; 0-{ (E-)-[2-(4-Chlorphenyl)-2-cyano-l-(2-trifluormethylphenyl)-vinyl] }S-Methylthiocarbonat; (E)-NI-[(2-Chlor-l,3-thiazol-5-ylmethyl)]-N2-cyano-NI- methylacetamidin; 1-(6-Chlorpyridin-3-ylmethyl)-7-methyl-8-nitro-1,2,3,5,6,7-hexahydro - imidazo[l,2-a]pyridin-5-ol; 4-[4-Chlorphenyl-(2-butylidin-hydrazono)methyl)]phenylmesylat; oder N-Ethyl-2,2-dichlor-l-methylcyclopropancarboxamide-2-(2,6-dichlor-trifluor-p-tolyl)hydrazon umfasst.

8. Verfahren zum Hemmen des Wachstums einer Alge, umfassend die folgenden Schritte:
Einführen der Zusammensetzung nach Anspruch 1 an einem Ort, an dem eine Hemmung erwünscht ist, in Mengen, die zur Hemmung der Algen wirksam sind.

9. Verfahren nach Anspruch 8, wobei der Ort eine Flüssigkeit ist, die ein Gewässer oder eine Farbe umfasst.

10. Verfahren nach Anspruch 8, wobei der Ort eine feste Fläche ist, ausgewählt aus der Gruppe bestehend aus Kunststoff, Beton, Holz, Glasfaser, Rohren aus Eisen und Polyvinylchlorid und Oberflächen, die mit Beschichtungsmaterialien und/oder Farbe bedeckt sind.

11. Verfahren nach Anspruch 8, wobei die Alge Chlamydomonas reinhardtii, Pesudoklrchneriella subacpitata, Scenedesmus quadricata, Anabaena sp., Oscillatoria tenuis, Microcystis aeruginosa, Navicula sp., Spirogyra sp. oder Char a sp. umfasst.

12. Verfahren zum Hemmen des Wachstums einer Alge, umfassend die folgenden Schritte:
Einführen der Zusammensetzung nach Anspruch 5 an einem Ort, an dem eine Hemmung erwünscht ist, in Mengen, die zur Hemmung der Algen wirksam sind.

13. Verfahren nach Anspruch 12, wobei der Ort eine Flüssigkeit ist, die ein Gewässer oder eine Farbe umfasst.

14. Verfahren nach Anspruch 12, wobei der Ort eine feste Fläche ist, ausgewählt aus der Gruppe bestehend aus Kunststoff, Beton, Holz, Glasfaser, Rohren aus Eisen und Polyvinylchlorid und Oberflächen, die mit Beschichtungsmaterialien und/oder Farbe bedeckt sind.

15. Verfahren nach Anspruch 12, wobei die Alge Chlamydomonas reinhardtii, Pesudoklrchneriella subacpitata, Scenedesmus quadricata, Anabaena sp., Oscillatoria tenuis, Microcystis aeruginosa, Navicula sp., Spirogyra sp. oder Char a sp. umfasst.

## Revendications

1. Composition algicide comprenant : un bouillon de cellules entières, un filtrat, un surnageant ou un extrait collecté à partir d'une fermentation de *Beauveria bassiana* 0379 (NRRL-67406), de *Brevibacillus brevis* HNYM3, 0989 (NRRL-B67408), de *Streptomyces spororaveus* F18, de P842 (NRRL-B67410), de *Paenibacillus elgii* SD17, de P858 (NRRL-B67407) ou de *Streptomyces* sp. Q004 (NRRL-67409).

2. Composition selon la revendication 1, comprenant en outre un support, un diluant, un adjuvant ou un tensioactif.

3. Composition selon la revendication 1, dans laquelle lesdites algues comprennent Chlamydomonas reinhardtii, Pesudokirchneriella subacpitata, Scenedesmus quadricata, Anabaena sp., Oscillatoria tenuis, Microcystis aeruginosa, Navicula sp., Spirogyra sp. ou Char a sp.

4. Composition selon la revendication 1, dans laquelle ladite composition est sous forme de poudres saupoudrables, de poudres solubles, de granulés solubles dans l'eau, de granulés dispersables dans l'eau, de poudres mouillables, de granulés, de concentrés solubles, de liquides miscibles à l'huile, de liquides à très faible volume, de concentrés émulsionnables, de concentrés dispersables, d'émulsions, de micro-émulsions, de concentrés en suspension, d'un concentré en suspension à base d'huile, d'aérosols, de brumisation/fumée, de suspensions en capsules ou d'une formulation de traitement des semences.

5. Composition selon la revendication 1, comprenant en outre un algicide chimique ou biologique.

6. Composition selon la revendication 5, dans laquelle ledit algicide biologique comprend Bacillus firmus, Bacillus thuringiensis, Bacillus pumilus, Bacillus licheniformis, Bacillus amyloliquefaciens, Bacillus subtilis, Paecilomyces lilacinus, Pasteuria penetrans, Pseudomonas sp., Brevabacillus sp., Lecanicillium sp., Ampelomyces sp., Pseudozyma sp., Streptomyces bikiniensis, Streptomyces costaricanus, Streptomyces avermitilis, Burkholderia sp., Trichoderma sp., Gliocladium sp., avermectine, Myrothecium sp., Paecilomyces spp., Sphingobacterium sp., Arthrobotrys sp., Chlorosplrnium, Neobulgaria, Daldinia, Aspergillus, Chaetomium, Lysobacter spp, Lachnum papyraceum, Verticillium suchlasporium, Arthrobotrys oligospora, Verticillium chlamydosporium, Hirsutella rhossiliensis, Pochonia chlamydosporia, Pleurotus ostreatus, Omphalotus olearius, Lampteromyces japonicas, Brevudimonas sp., ou Muscodor sp.

7. Composition selon la revendication 5, dans laquelle ledit algicide chimique comprend de la 3-(4-chloro-2,6-diméthylphényl)-4-hydroxy-8-oxa-I-azaspiro[4,5]déc-3-en-2-one ; de la 3-(4'-chloro-2,4- diméthyl[l,l'-biphényl]-3-yl)-4-hydroxy-8-oxa-1-azaspiro-[4,5]déc-3-en- 2-one ; de la 4-[[(6-chloro-3-pyridinyl)méthyl]méthylamino]-2(5H)-furanone ; de la 4-[[(6-chloro-3-pyridinyl)méthyl]cyclopropylamino]-2(5H)-furanone ; du 3-chloro-N2-[(IS)-l-méthyl-2-(méthylsulfonyl)éthyl]-N1-[2-méthyl-1-4-[1,2,2,2-tétrafluoro-1 -(trifluorométhyl)éthyl]phényl]-1,2-benzènedicarboxamide ; 2-cyano-N-éthyl-4-fluoro-3-méthoxy-bènesulfonamide ; du 2-cyano-N-éthyl-3-méthoxy-benzènesulfonamide ; du 2-cyano-3-difluorométhoxy-N-éthyl-4-fluoro-benzènesulfonamide ; du 2-cyano-3-fluorométhoxy-N-éthylbenzènesulfonamide ; du 2-cyano-6-fluoro-3-méthoxy-N,N-diméthyl-benzènesulfonamide; du 2-cyano-N-éthyl-6-fluoro-3-méthoxy-N-méthyl-benzènesulfonamide ; du 2-cyano-3-difluorométhoxy-N,N-diméthylbenzènesulfonamide ; du 3-(difluorométhyl)-N-[2-(3,3-diméthylbutyl)phényl]-1-méthyl-IH-pyrazole-4-carboxamide ; de la N-éthyl-2,2-diméthylpropionamide-2-(2,6-dichloro-D,D,D,-trifluoro-p-tolyl)hydrazone ; de la N-éthyl-2,2-dichloro-l-méthylcyclopropane-carboxamide-2-(2,6-dichloro-D,D,D,-trifluoro-p-tolyl)hydrazone nicotine ; du thiocarbonate de 0-{(E-)-[2-(4-chloro-phényl)-2-cyano-l-(2-trifluorométhylphényl)-vinyl] }S-méthyle ; de la (E)-NI-[(2-chloro-1,3-thiazol-5-ylméthyl)]-N2-cyano-NI-méthylacétamidine ; du l-(6-chloropyridin-3-ylméthyl)-7-méthyl-8-nitro-1,2,3,5,6,7-hexahydro-imidazo[1,2-a]pyridin-5-ol ; du mésylate de 4-[4-chlorophényl-(2-butylidine-hydrazono)méthyl)]phényle ; ou de la N-éthyl-2,2-dichloro-l-méthylcyclopropanecarboxamide-2-(2,6-dichloro-trifluoro-p-tolyl)hydrazone.

8. Procédé d'inhibition de la croissance d'une algue comprenant les étapes de :
introduction de la composition selon la revendication 1 dans un emplacement où l'inhibition est souhaitée, en des quantités efficaces pour inhiber lesdites algues.

9. Procédé selon la revendication 8, dans lequel ledit emplacement est un liquide comprenant une masse d'eau ou de la peinture.

10. Procédé selon la revendication 8, dans lequel ledit emplacement est une surface solide choisie dans le groupe constitué du plastique, du béton, du bois, de la fibre de verre, des tuyaux en fer et en poly(chlorure de vinyle), et des surfaces recouvertes de matériaux de revêtement et/ou de peinture.

11. Procédé selon la revendication 8, dans lequel lesdites algues comprennent Chlamydomonas reinhardtii, Pesudokirchneriella subacpitata, Scenedesmus quadricata, Anabaena sp., Oscillatoria tenuis, Microcystis aeruginosa, Navicula sp., Spirogyra sp. ou Char a sp.

12. Procédé d'inhibition de la croissance d'une algue comprenant les étapes de :
introduction de la composition selon la revendication 5 dans un emplacement où l'inhibition est souhaitée, en des quantités efficaces pour inhiber lesdites algues.

13. Procédé selon la revendication 12, dans lequel ledit emplacement est un liquide comprenant une masse d'eau ou de la peinture.

14. Procédé selon la revendication 12, dans lequel ledit emplacement est une surface solide choisie dans le groupe constitué du plastique, du béton, du bois, de la fibre de verre, des tuyaux en fer et en poly (chlorure de vinyle), et des surfaces recouvertes de matériaux de revêtement et/ou de peinture.

15. Procédé selon la revendication 12, dans lequel lesdites algues comprennent Chlamydomonas reinhardtii, Pesudokirchneriella subacpitata, Scenedesmus quadricata, Anabaena sp., Oscillatoria tenuis, Microcystis aeruginosa, Navicula sp., Spirogyra sp. ou Char a sp.
